# EUROPEAN PATENT SPECIFICATION

(11) **EP 3 765 853 B1**
(45) Date of publication and mention of the grant of the patent: **01.05.2024**
(21) Application number: 19713958.7
(22) Date of filing: 13.03.2019
(51) Int. Cl.: G01N 33/574

(54) **METHODS FOR MONITORING TREATMENT RESPONSE AND DISEASE PROGRESSION IN SUBJECTS USING CIRCULATING CELLS**
VERFAHREN ZUR ÜBERWACHUNG DER BEHANDLUNGSREAKTION UND DES KRANKHEITSVERLAUFS IN SUBJEKTEN UNTER VERWENDUNG ZIRKULIERENDER ZELLEN
PROCÉDÉS DE SURVEILLANCE DE LA RÉPONSE AU TRAITEMENT ET DE LA PROGRESSION D'UNE MALADIE CHEZ DES SUJETS À L'AIDE DE CELLULES CIRCULANTES

(30) Priority: 13.03.2018 US 201862642318 P; 16.04.2018 US 201862658122 P; 19.10.2018 US 201862747796 P; 16.11.2018 US 201862768561 P; 19.01.2019 US 201962794580 P
(43) Date of publication of application: 20.01.2021
(73) Proprietor: Creatv Microtech, Inc., Potomac, MD 20854 (US)
(72) Inventor: ADAMS, Daniel, Basking Ridge, NJ 07920 (US); TANG, Cha-Mei, Potomac, MD 20854 (US)
(74) Representative: Fuchs Patentanwälte Partnerschaft mbB
(86) International application number: PCT/US2019/022050
(87) International publication number: WO 2019/178226

(56) References cited:
- WO-A1-2013/181532
- WO-A1-2016/033103
- WO-A1-2017/181073
- WO-A1-2018/151865
- WO-A1-2018/184005
- YONG TANG ET AL: "Biomarkers for early diagnosis, prognosis, prediction, and recurrence monitoring of non-small cell lung cancer", ONCOTARGETS AND THERAPY, vol. Volume 10, 1 September 2017 (2017-09-01), pages 4527-4534, XP055594458, DOI: 10.2147/OTT.S142149
- D. L. ADAMS ET AL: "Circulating Cancer-Associated Macrophage-Like Cells Differentiate Malignant Breast Cancer and Benign Breast Conditions", CANCER EPIDEMIOLOGY, BIOMARKERS AND PREVENTION., vol. 25, no. 7, 1 July 2016 (2016-07-01), pages 1037-1042, XP055479663, US ISSN: 1055-9965, DOI: 10.1158/1055-9965.EPI-15-1221
- DANIEL L. ADAMS ET AL: "Circulating giant macrophages as a potential biomarker of solid tumors", PROCEEDINGS OF THE NATIONAL ACADEMY OF SCIENCES OF THE UNITED STATES OF AMERICA, vol. 111, no. 9, 18 February 2014 (2014-02-18), pages 3514-3519, XP055248775, US ISSN: 0027-8424, DOI: 10.1073/pnas.1320198111
- DANIEL L ADAMS ET AL: "Abstract 2631: Cancer associated macrophage-like (CAMLs) cells in blood predict progression and survival for all stages of solid tumors", PROCEEDINGS: AACR ANNUAL MEETING, 14 April 2018 (2018-04-14), XP055594339, DOI: 10.1158/1538-7445.AM2018-2631Published
- DANIEL L ADAMS ET AL: "Combining circulating tumor cells (CTCs) and circulating cancer associated macrophage-like cells (CAMLs) for accurately predicting responsiveness of new line therapies in late stage cancers", CLINICAL ONCOLOGY, vol. 36, no. NO. 15_SUPPL, 1 June 2018 (2018-06-01), XP055594350,

## Description

### FIELD OF THE INVENTION

The present invention generally relates methods of monitoring treatment response and disease progression in subjects having cancer, such as a solid tumor, using circulating cell biomarkers in the blood and other bodily fluids.

### RELATED ART

When tumor cells break away from primary solid tumors, they penetrate into the blood or lymphatic circulation, and ultimately leave the blood stream and enter either organs or tissue to form metastasis. 90% of cancer-related deaths are caused by the metastatic process. The most common metastatic sites are the lung, liver, bone and brain. Tumor cells found in the circulation are called circulating tumor cells (CTCs). Many research publications and clinical trials show that CTCs have clinical utility in (i) providing prognostic survival and cancer recurrence information through the enumeration of CTCs in the blood stream, and (ii) providing treatment information through examination of protein expression levels, and the occurrence of gene mutations and translocations in the CTCs. However, CTCs are not consistently associated with the development and/or presence of cancer in a subject, even in stage IV cancer patients. While CTCs are found most often in stage IV of breast, prostate and colorectal cancers, they are rare in early stages of the same cancer. CTCs are also rare in other cancers.

Circulating cancer associated macrophage-like cells (CAMLs) are another cancer-related cell type that is found in the blood of subjects having cancer. They are circulating giant stromal cells. CAMLs are associated with all solid tumors tested and all stages of cancer. CAMLs are polyploid and very large in size, ~25 µm to ~300 µm in size. These polyploid cells can be either CD45(-) or CD45(+) and most of the time express CD1 1c, CD14 and CD31, which confirms their origin as a myeloid lineage. They are often found in the process of engulfing CTCs and cell debris [1,6].

Assays associated with the identification and characterization of biomarkers, such as CAMLs, in blood and other body fluids can be used to provide a variety of information, including indications as to whether a disease will progress in a subject having cancer, whether a subject having cancer is responding to treatment, whether a subject having cancer is likely to respond to future treatment, and overall survival (OS) and progression free survival (PFS) information in such subjects. The present invention is directed to providing such tools to clinicians for predicting disease progression and treatment response and other important goals.

The importance of being able to predict disease progression and treatment response by a blood test quickly is associated with providing the opportunity to change the treatment if the first treatment is not effective. To able to make a change quickly serves to minimize the cancer progression before another treatment. For immunotherapy, quick switch also reduces cost, because immunotherapy can cost between $150,000 to greater than $350,000 a year. Yong Tang et al.: "Biomarkers for early diagnosis, prognosis, prediction, and recurrence monitoring of non-small cell lung cancer", ONCOTARGETS AND THERAPY, vol. Volume 10, 1 September 2017 (2017-09-01), reviews a wide variety of biomarkers for predicting cancer progression and tratment response.

### SUMMARY

The present invention is directed methods of using a type of cell with unique characteristics that is found in the blood of subjects having solid tumors, including carcinoma, sarcoma, neuroblastoma and melanoma. These circulating cells, termed "circulating Cancer Associated Macrophage-like cells" (CAMLs), have been shown to be associated with the presence of solid tumors in a subject having cancer. Five morphologies associated with CAMLs have been characterized and described [1,2]. CAMLs have been found by various methods in the peripheral blood of cancer patients. Size exclusion methodology, such as microfiltration using a precision microfilter, provides consistent isolation of CAMLs from the peripheral blood of subjects having stage I to stage IV solid tumors.

Medical applications associated with CAMLs include, but are not limited to, use of the cells as a biomarker to provide early detection of cancer and diagnosis of cancer, in particular in the early detection and diagnosis of cancer relapse or recurrence, and in the determination of cancer mutation. CAMLs are shown to have clinical utility as a biomarker in making prognosis regarding patient survival. CAMLs are also shown through the data presented herein to have clinical utility as a biomarker in making predictions of treatment response and disease progression.

Thus, the present invention is directed to methods for predicting disease progression and/or treatment response in subjects having cancer, such as a solid tumor cancer.

In a first embodiment, the methods of the invention comprise determining the size of circulating cells, such as CAMLs, in two or more biological samples obtained from a subject having cancer over time, wherein when a decrease in size of the circulating cells between the samples is found over time, cancer is predicted not to progress and/or the subject is found to be responding to treatment. In related embodiment, the methods of the invention comprise determining the number of circulating cells, such as CAMLs, in two or more biological samples obtained from a subject having cancer over time, wherein when a decrease in the number of the circulating cells between the samples is found over time, cancer is predicted not to progress and/or the subject is found to be responding to treatment.

In a related aspect, the method comprises determining the size of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the size of at least one cell in the first sample is about 50 µm or more, and the size of each cell in the second and optional additional samples is less than about 50 µm, cancer is predicted not to progress and/or the subject is found to be responding to treatment.

In another related aspect, the method comprises determining the average size of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the average size of circulating cells in the second and optional additional samples is decreased in comparison to the average size of the cells in the first sample, the subject is identified as responding to treatment. In some aspects, the average size of the circulating cells in the first sample is about 50 µm or more.

In a second embodiment, the invention is directed to methods for predicting cancer progression in a subject having cancer comprising determining the size of circulating cells, such as CAMLs, in a biological sample obtained from a subject having cancer, and making a prediction based thereon, wherein when each circulating cell in the sample is less than about 50 µm in size, the cancer is predicted not to progress, and wherein when at least one circulating cell in the sample is about 50 µm or more in size, the cancer is predicted to progress.

In one aspect of the embodiment, the invention is directed to methods for predicting cancer progression in a subject having a cancer comprising determining the size of circulating cells, such as CAMLs, in a first and second biological sample, and optional additional biological samples, obtained from a subject having cancer, wherein the first sample is obtained from the subject prior to or during cancer treatment, wherein the second sample and optional additional samples are obtained from the subject after at least one cancer treatment,
wherein when the average size of CAMLs in the second and optional additional samples is decreased in comparison to the average size of the CAMLs in the first sample, the cancer is predicted not to progress in the subject and the subject is optionally identified as responding to treatment; or
wherein when the average size of CAMLs in the second and optional additional samples is maintained or increased in comparison to the average size of the CAMLs in the first sample, the cancer is predicted to progress in the subject and the subject is optionally identified as not responding to treatment; or
wherein when the size of at least one CAML in the first sample is about 50 µm or more, and the size of each cell in the second and optional additional samples is less than about 50 µm, the cancer is predicted not to progress and the subject is optionally identified as responding to treatment; or
wherein when the size of each CAML in the first sample is less than about 50 µm, and when at least one CAML in the second and optional additional samples is greater than about 50 µm in size, the cancer is predicted to progress and the subject is optionally identified as not responding to treatment. In some aspects, the average size of the circulating cells in the first sample is about 50 µm or more.

In another aspect of the embodiment, the invention is directed to methods for predicting cancer progression in a subject having a cancer comprising determining the number of circulating cells, such as CAMLs, in a first and second biological sample, and optional additional biological samples, obtained from a subject having cancer, wherein the first sample is obtained from the subject prior to or during cancer treatment, wherein the second sample and optional additional samples are obtained from the subject after at least one cancer treatment, wherein when the number of circulating cells in the second and optional additional samples is decreased in comparison to the number of the circulating cells in the first sample, the cancer is predicted not to progress and the subject is optionally identified as responding to treatment, and wherein when the number of circulating cells in the second and optional additional samples is maintained or increased in comparison to the number of the circulating cells in the first sample, the cancer is predicted to progress and the subject is optionally identified as not responding to treatment.

In a third embodiment, the invention is directed to methods for predicting response to treatment of a subject having cancer comprising determining the size of circulating cells, such as CAMLs, in a biological sample obtained from the subject, and making a prediction based thereon, wherein when each circulating cell in the sample is less than about 50 µm in size, the subject is predicted to respond to treatment, and wherein when at least one circulating cell in the sample is about 50 µm or more in size, the subject is predicted to not respond to treatment.

In one aspect of the embodiment, the invention is directed to methods for predicting response to treatment of a subject having cancer comprising determining the size of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, and
wherein when the average size of CAMLs in the second and optional additional samples is decreased in comparison to the average size of the CAMLs in the first sample, the subject is predicted to respond to treatment;
wherein when the average size of CAMLs in the second and optional additional samples is maintained or increased in comparison to the average size of the CAMLs in the first sample, the subject is predicted to not respond to treatment;
wherein when the number of circulating cells greater than about 50 µm in size is maintained or increases from the first to the second and optional additional samples, the subject is predicted to not respond to treatment; or
wherein when the number of circulating cells greater than about 50 µm in size decreases from the first to the second and optional additional samples, the subject is predicted to respond to treatment; or
wherein when the size of each circulating cell in the second or following sample decreases in comparison to the first sample, and wherein the size of each circulating cell in the second or optional additional sample is less than about 50 µm, the subject is predicted to respond to treatment and potentially cured of cancer.

In another aspect of the embodiment, the invention is directed to methods for predicting response to treatment of a subject having cancer comprising determining the absence of any circulating cells, such as CAMLs, larger than about 50 µm in a biological sample from the subject and predicting the subject is responding to treatment.

In a further aspect of the embodiment, the invention is directed to methods for predicting response to treatment of a subject having cancer comprising determining the size of circulating cells, such as CAMLs, in a biological sample obtained from a subject having cancer, wherein the sample is obtained from the subject after at least one cancer treatment, and wherein when each circulating cell is about 50 µm or less in size, the subject is predicted to respond to treatment.

In an additional aspect of the embodiment, the invention is directed to methods for predicting response to treatment of a subject having cancer comprising determining the number of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the number of circulating cells decreases from the first to the second and optional additional samples, the subject is predicted to respond to treatment, and wherein when the number of circulating cells is maintained or increases from the first to the second and optional additional samples, the subject is predicted to not respond to treatment.

In a fourth embodiment, the invention is directed to methods predicting resistance to treatment in a subject having lung cancer, said method comprising determining the size of circulating cells, such as CAMLs, in a biological sample from a subject having cancer, wherein when at least one cell in said sample is about 50 µm or more in size, the subject is predicted to be more resistant to cancer treatment than a subject having cancer where none of the cells is more than about 50 µm in size. In certain aspects of this embodiment, the cancer is lung cancer.

The present invention is also directed to methods of treating cancer, where treatment decisions can be based on predictions of treatment response using the circulating cells in the methods described above.

For example, and in a fifth embodiment, the invention is directed to methods of treating a subject having cancer comprising administering a therapeutically effective amount of a cancer treatment to a subject having cancer and determining the size of circulating cells, such as CAMLs, in two or more biological samples obtained from a subject having cancer, wherein a first biological sample is obtained from the subject before or during the cancer treatment, wherein a second biological sample is obtained from the subject during or after the cancer treatment, wherein when a decrease in size of the circulating cells between the samples is found over time, the subject is found to be responding to treatment and the treatment is continued, and wherein when a decrease in size of the circulating cells between the samples is not found over time, the subject is found not to be responding to treatment and the treatment is not continued.

In related embodiment, the methods of the invention comprise administering a therapeutically effective amount of a cancer treatment to a subject having cancer and determining the number of circulating cells, such as CAMLs, in two or more biological samples obtained from a subject having cancer, wherein a first biological sample is obtained from the subject before or during the cancer treatment, wherein a second biological sample is obtained from the subject during or after the cancer treatment, wherein when a decrease in the number of the circulating cells between the samples is found over time, the subject is found to be responding to treatment and the treatment is continued, and wherein when a decrease in the number of the circulating cells between the samples is not found over time, the subject is found not to be responding to treatment and the treatment is not continued.

In an aspect of this embodiment, the method comprises administering a therapeutically effective amount of a cancer treatment to a subject having cancer and determining the size of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, wherein when the size of at least one cell in the first sample is about 50 µm or more, and the size of each cell in the second and optional additional samples is less than about 50 µm, the subject is found to be responding to treatment and the treatment is continued.

In another related aspect, the method comprises administering a therapeutically effective amount of a cancer treatment to a subject having cancer and determining the average size of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, wherein when the average size of circulating cells in the second and optional additional samples is decreased in comparison to the average size of the cells in the first sample, the subject is identified as responding to treatment and the treatment is continued, and wherein when the average size of circulating cells in the second and optional additional samples is not decreased in comparison to the average size of the cells in the first sample, the subject is identified as not responding to treatment and the treatment is not continued. In some aspects, the average size of the circulating cells in the first sample is about 50 µm or more.

In a sixth embodiment, the invention is directed to methods of treating a subject having cancer comprising administering a therapeutically effective amount of a cancer treatment to a subject having cancer and determining the size of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, and
wherein when the average size of CAMLs in the second and optional additional samples is decreased in comparison to the average size of the CAMLs in the first sample, the subject is predicted to respond to treatment and the treatment is continued;
wherein when the average size of CAMLs in the second and optional additional samples is maintained or increased in comparison to the average size of the CAMLs in the first sample, the subject is predicted to not respond to treatment and the treatment is not continued;
wherein when the number of circulating cells greater than about 50 µm in size is maintained or increases from the first to the second and optional additional samples, the subject is predicted to not respond to treatment and the treatment is not continued;
wherein when the number of circulating cells greater than about 50 µm in size decreases from the first to the second and optional additional samples, the subject is predicted to respond to treatment and the treatment is continued; or
wherein when the size of each circulating cell in the second or following sample decreases in comparison to the first sample, and wherein the size of each circulating cell in the second or optional additional sample is less than about 50 µm, the subject is predicted to respond to treatment and the treatment is continued.

In an aspect of the embodiment, the invention is directed to methods of treating a subject having cancer comprising administering a therapeutically effective amount of a cancer treatment to a subject having cancer and determining the absence of any circulating cells, such as CAMLs, larger than about 50 µm in a biological sample obtained from the subject, wherein the sample is obtained from the subject after at least one cancer treatment, and wherein when there is an absence of any circulating cells larger than about 50 µm in the biological sample the subject is predicted to respond to treatment and the treatment is continued.

In a further aspect of the embodiment, the invention is directed to methods of treating a subject having cancer comprising administering a therapeutically effective amount of a cancer treatment to a subject having cancer and determining the size of circulating cells, such as CAMLs, in a biological sample obtained from the subject, wherein the sample is obtained from the subject after at least one cancer treatment, and wherein when each circulating cell is about 50 µm or less in size, the subject is predicted to respond to treatment and the treatment is continued.

In an additional aspect of the embodiment, the invention is directed to methods of treating a subject having cancer comprising administering a therapeutically effective amount of a cancer treatment to a subject having cancer and determining the number of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, wherein when the number of circulating cells decreases from the first to the second and optional additional samples, the subject is predicted to respond to treatment and the treatment is continued, and wherein when the number of circulating cells is maintained or increases from the first to the second and optional additional samples, the subject is predicted to not respond to treatment and the treatment is not continued.

In each of these embodiment and aspects of the present invention, if no circulating cells are found in the biological sample at the end of the treatment, it may be concluded that the cancer has been eliminated. When circulating cells are still found in the biological sample at the end of the treatment, it may be concluded that the cancer in the patient has not been eliminated, and that the patient has residual disease.

The aggressiveness of residual disease can be predicted by the size of the circulating cells. For example, cancer progresses faster when at least one CAMLs in a biological sample is 50 um in size or more.

In each of the embodiments and aspects of the invention, the circulating cells may be CAMLs. CAMLs are defined as having each of the following characteristics:
(a) large atypical polyploid nucleus of about 14-64 µm in size, multiple individual nuclei and/or one or more fused nuclei having a size of about 14-64 µm;
(b) cell size of about 20-300 µm in size; and
(c) morphological shape selected from the group consisting of spindle, tadpole, round, oblong, two legs, more than two legs, thin legs, and amorphous.

In certain aspects of the embodiments of the invention, the circulating cells can be further defined as having one or more of the following additional characteristics:
(d) CD14 positive phenotype;
(e) CD45 expression;
(f) EpCAM expression;
(g) vimentin expression;
(h) PD-L1 expression;
(i) CD11C marker expression;
(j) CD146 marker expression;
(k) CD202b marker expression;
(l) CD31 marker expression; and
(m) CK8, 18, 19 epithelial phenotype.

In each of the embodiments and aspects of the invention, the size of the biological sample is between 0.5 and 50 mL. The size of the biological sample may also be between 5 and 15 mL. In certain aspects of the invention, the size of the biological sample is about 7.5 mL.

In each of the embodiments and aspects of the invention, the source of the biological sample is one or more of blood, lymph node, bone marrow, cerebral spinal fluid, tissue, urine, peripheral blood mononuclear cells (PBMCs), and cryopreserved PBMCs. When the biological sample is blood, the blood may be peripheral blood, antecubital-vein blood, inferior-vena-cava blood, femoral vein blood, portal vein blood, or jugular-vein blood, for example. The sample may be a fresh sample or a properly prepared cryo-preserved sample that is thawed.

In certain aspects of the embodiments of the invention, the cancer is a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer.

In certain aspects of the embodiments of the invention, the circulating cells are isolated from the biological samples using one or more means selected from size exclusion methodology, immunocapture, dendrimer-mediated multivalent cell capture, affinity based surface capture, biomimetic surface coating capture, selectin coated surfaces capture, other functionalized surface captures, inertial focusing chips, red blood cell lysis, white blood cell depletion, FICOLL separation, electrophoresis, dielectrophoresis, flow cytometry, magnetic levitation, and various microfluidic chips, or a combination thereof.

The term "micron" mentioned in this and other paragraphs shall be regarded as pertaining to the SI unit "µm". In one aspect of the invention, circulating cells are isolated from the biological samples using size exclusion methodology that comprises using a microfilter. The microfilter may have a pore size ranging from about 5 microns to about 20 microns. Suitable ranges of pore sizes also include, but are not limited to, pore sizes ranging from about 5-7 microns, about 7-8 microns, 8-10 microns, 11-13 microns, 14-16 microns, 17-20 microns, 5-10 microns, 11-15 microns, 15-20 microns, 9-15 microns, 16-20 microns, and 9-20 microns. Another suitable range comprises pore sizes ranging from about 5-20 microns, but excluding pores of between 7 and 8 microns. The pores of the microfilter may have a round, race-track shape, oval, slit, square, rectangular and/or other shapes. The microfilter may have precision pore geometry, uniform pore distribution, more than one pore geometry, and/or non-uniform pore distribution. The microfilter may be single layer, or multi-layers with different shapes on different layers. Microfilters having round pores of about 7-8 microns in size are especially optimal when polymeric microfilters are used. In a preferred aspect, the microfilter has precision pore geometry and uniform pore distribution.

In another aspect of the invention, circulating cells are isolated from the biological samples using a CellSieve^{™} low-pressure microfiltration assay.

In another aspect of the invention, circulating cells are isolated from the biological samples using a microfluidic chip via physical size-based sorting, slits, channels, hydrodynamic size-based sorting, grouping, trapping, immunocapture, concentrating large cells, or eliminating small cells based on size.

In certain aspects of the embodiments of the invention, the subject is undergoing treatment. The treatment may be one or more of chemotherapy, single drug, combination of drugs, immunotherapy, radiation therapy, chemoradiation, radiation combined with single or multiple drug, chemoradiation combined with single or multiple drugs, cancer vaccine, and cell therapy. When the treatment is a cancer vaccine, the subject may express at least one HLA allele.

In each of the embodiments and aspects of the invention, CAMLS may be used independently as a cancer marker, or in combination with other circulating cells, such as circulating tumor cells (CTCs), epithelial mesenchymal transition calls (EMTs), and circulating cancer associated vascular endothelial cells (CAVEs), as well as with cell-free DNA (cfDNA), circulating tumor DNA (ctDNA), methylated DNA, proteomic, metabolomic, lipidomic and other biomarkers to provide a more complete understanding of a patient's disease. CAMLs are the only cell type among the group of circulating cells mentioned herein that is typically larger than 30 um in size.

When the methods of the invention are associated with predicting treatment responses in a subject, the time scale to obtain treatment response information can be as short as two treatment cycles or 60 days, depending on the type of treatment.

In the relevant aspects of the invention, the test to predict cancer treatment response may be one or more of the following:
- independent of the type of solid tumor,
- independent of the stage of cancer,
- independent of the type of treatment,
- at the start of treatment, during ongoing treatment or at end of treatment,
- based on blood sample,
- not requiring tissue, and
- made via determination obtained earlier than imaging.

The subjects mentioned in the methods of the present invention will be a human, a non-human primate, bird, horse, cow, goat, sheep, a companion animal, such as a dog, cat or rodent, or other mammal.

### BRIEF DESCRIPTION OF THE DRAWINGS

**Figures 1A-1D** show the changes of size of the largest CAML and its relationship to response for n=42 non-small cell lung cancer patients treated by radiation. Dashed lines indicate responders and solid lines indicate non-responders. Response is determined at 24 months.
**Figures 2A-2C** show the Kaplan Meier plots of progression free survival (PFS) at three time points, (Fig. 2A) baseline before chemoradiation, (Fig. 2B) first follow up (~ day 30 after baseline), and (Fig. 2C) second follow up (~ 60 days after baseline) analyzed based on the size of the CAMLs for lung and esophageal cancer patients treated by radiation.
**Figures 3A-3C** show the Kaplan Meier plots of overall survival (OS) at three time points, (Fig. 3A) baseline before chemoradiation, (Fig. 3B) first follow up (~ day 30 after baseline), and (Fig. 3C) second follow up (~ 60 days after baseline) analyzed based on the size of the CAMLs for lung and esophageal cancer patients treated by radiation.
**Figures 4A-4F** show the size of the largest CAML in breast, prostate and lung cancer patients that were (Fig. 4A) below 50 µm at baseline and also below 50 µm at the second follow up, (Fig. 4B) below 50 µm at baseline and changed to above 50 µm at the second follow up, (Fig. 4C) above 50 µm at baseline and changed to below 50 µm at the second follow up, (Fig. 4D) above 50 µm at baseline and also above 50 µm at the second follow up, (Fig. 4E) below 50 µm at second follow up independent the size at baseline, and (Fig. 4F) above 50 µm at second follow up independent the size at baseline.
**Figures 5A-5B** show the Kaplan Meier plots of progression free survival (PFS) at two time points, (Fig. 5A) baseline before therapy and (Fig. 5B) follow up (~ day 90 after baseline) analyzed based on the size of the CAMLs for breast, prostate and lung cancer patients.
**Figures 6A-6B** show the Kaplan Meier plots of overall survival (OS) at two time points, (Fig. 6A) baseline before therapy and (Fig. 6B) follow up (~ day 90 after baseline) analyzed based on the size of the CAMLs for breast, prostate and lung cancer patients.
**Figure 7** shows that CTCs were identified in 21% of patients at BL and 23% at follow-up. CTCs are found more often in breast cancer, but not in other types of cancers. In contrast, CAMLs are found in high percentages in all 5 types of cancers.
**Figures 8A-8C** show the Kaplan Meier plot of overall survival (OS) at FU for (Fig. 8A) with CTC versus no CTCs, (Fig. 8B) CAML size < 50 µm versus ≥ 50 µm, and (Fig. 8C) for ≥ 1 CTC or ≥ 50 µm versus 0 CTC or < 50 µm.
**Figure 9** shows the intensity of cell differentiation markers used to identify and subtype CAMLs.
**Figure 10** shows the number of CAMLs for 10 breast cancer patients over time undergoing vaccine treatment.
**Figure 11** shows the size of CAMLs for 10 breast cancer patients over time undergoing vaccine treatment.

### DETAILED DESCRIPTION

As used herein, "a" or "an" may mean one or more. As used herein when used in conjunction with the word "comprising," the words "a" or "an" may mean one or more than one. As used herein "another" may mean at least a second or more. Furthermore, unless otherwise required by context, singular terms include pluralities and plural terms include the singular.

As used herein, "about" refers to a numeric value, including, for example, whole numbers, fractions, and percentages, whether or not explicitly indicated. The term "about" generally refers to a range of numerical values (e.g., +/- 5, 6, 7, 8, 9 or 10% of the recited value) that one of ordinary skill in the art would consider equivalent to the recited value (e.g., having the same function or result). In some instances, the term "about" may include numerical values that are rounded to the nearest significant figure.

The matters defined in the description such as a detailed construction and elements are nothing but the ones provided to assist in a comprehensive understanding of the invention.

Cancer is one of the most feared illness in the world, affecting all populations and ethnicities in all countries. Approximately 40% of both men and women will develop cancer in their lifetime. In the United States alone, at any given time there are more than 12 million cancer patients, with 1.7 million new cancer cases and more than 0.6 million deaths estimated for 2018. Cancer death worldwide is estimated to be about 8 million annually, of which 3 million occur in developed countries where patients have access to treatment.

Ideally, there will be diagnostics that can quickly determine if a selected therapy is working and provide prognosis for disease progression.

In this disclosure, a cell type is presented that is more consistently found in the blood of solid tumor patients from Stages I-IV than any other cancer related cell. These circulating cells are macrophage-like cells that contain the same tumor markers as the primary tumor and they are termed circulating Cancer Associated Macrophage-like cells (CAMLs) herein.

Along with circulating tumor cells (CTCs), CAMLs present in biological samples from patients having cancer can be isolated and characterized, for example through the use of size exclusion methods, including microfiltration methods. Microfilters can be formed with pores large enough to allow red blood cells and the majority of white blood cells to pass, while retaining larger circulating cells, such as CTCs and CAMLs. The collected cells can then be characterized, either directly on the filters or through other means.

CAMLs have many clinical utilities when used alone. However, the characterization of CAMLs in a biological sample using the methods of the present invention can be combined with the assaying of other markers for the same characteristics (e.g., size and number) or other characteristics. These other markers include CTCs, epithelial mesenchymal transition calls (EMTs), and circulating cancer associated vascular endothelial cells (CAVEs), as well as with cell-free DNA (cfDNA), circulating tumor DNA (ctDNA), methylated DNA, proteomic, metabolomic, lipidomic and other biomarkers, and free proteins in blood to further improve sensitivity and specificity of a method defined herein. This is especially true for CAMLs and CTCs because they can be isolated and identified at the same time using the same methods disclosed herein.

### Circulating Cancer Associated Macrophage-like Cells (CAMLs)

As defined herein, CAMLs have one or more of the following features:
- CAMLs have a large, atypical polyploid nucleus or multiple individual nuclei, often scattered in the cell, though enlarged fused nucleoli are common. CAML nuclei generally range in size from about 10 µm to about 70 µm in diameter, more commonly from about 14 µm to about 64 µm in diameter.
- For many cancers, CAMLs express the cancer marker of the disease. For example, CAMLs associated with epithelial cancers may express CK 8, 18 or 19, vimentin, etc. The markers are typically diffused, or associated with vacuoles and/or ingested material. The staining pattern for any marker is nearly uniformly diffused throughout the whole cell. For sarcomas, neuroblastomas and melanomas, other markers associated with the cancers can be used instead of CK 8, 18, 19.
- CAMLs can be CD45 positive or CD45 negative, and the present invention encompasses the use of both types of CAMLs.
- CAMLs are large, approximately 20 micron to approximately 300 micron in size by the longest dimension.
- CAMLs are found in many distinct morphological shapes, including spindle, tadpole, round, oblong, two legs, more than two legs, thin legs, or amorphous shapes.
- CAMLs from carcinomas typically have diffused cytokeratins.
- If CAMLs express EpCAM, EpCAM is typically diffused throughout the cell, or associated with vacuoles and/or ingested material, and nearly uniform throughout the whole cell, but not all CAML express EpCAM, because some tumors express very low or no EpCAM.
- If CAMLs express a marker, the marker is often diffused throughout the cell, or associated with vacuoles and/or ingested material, and nearly uniform throughout the whole cell, but not all CAML express the same markers with equal intensity and for a limited number of markers, the markers are not distributed equally throughout the cell.
- CAMLs often express markers associated with the markers of the tumor origin, e.g. if the tumor is of prostate cancer origin and expresses PSMA, then CAMLs from such a patient also expresses PSMA. As another example, if the primary tumor is of pancreatic origin and expresses PDX-1, then CAMLs from such a patient also expresses PDX-1. As further example, if the primary tumor or CTC of the cancer origin express CXCR-4, then CAMLs from such a patient also express CXCR-4.
- If the primary tumor or CTC originating from the cancer expresses a biomarker of a drug target, CAMLs from such a patient also express the biomarker of the drug target. An example of such a biomarker of immunotherapy is PD-L1.
- CAMLs express monocytic markers (e.g. CD11c, CD14) and endothelial markers (e.g. CD146, CD202b, CD31).
- CAMLs have the ability to bind Fc fragments.

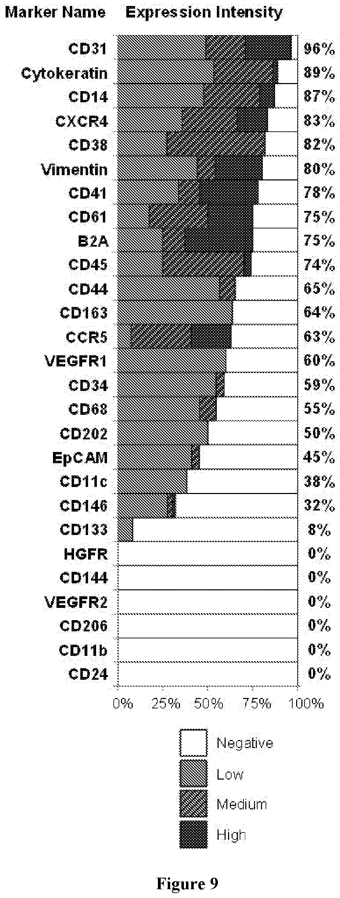

An extensive set of markers were evaluated for their expression on CAMLs, and the results are shown in **FIG. 9****.** In one aspect of the invention, the CAMLs of the present invention express 1, 2, 3, 4, 5, 6, 7, 8, 9, 10, 11, 12, 13, 14, 15, 16, 17, 18, 19, 20 or all 21 of the markers shown in **FIG. 9****.** The markers were screened against 1118 CAMLs from 93 different patients with different cancers.

The markers, and thus the CAMLs themselves, can be detected and/or characterized using various means known to the skilled artisan. For example, antibodies having binding specificity for particular markers can be used to select, detect and/or identify cells expressing one or more markers. CAMLs were initially isolated and identified with DAPI, cytokeratin, and CD45; then sequentially re-stained with total of 27 markers including myeloid/macrophage, white blood cell, megakaryocyte, epithelial, endothelial, progenitor/stem, and motility markers. As can be seen from **FIG. 9****,** marker expression ranges from 0% to 96%. Almost all CAMLs were found to express levels of CD31, and commonly co-expressed cytokeratin, CD14, CXCR4, vimentin and other markers. However, while CAMLs contained clear myeloid lineage marker (CD14), CD31 marker was expressed more often at 96%.

CAMLs also present with numerous phenotypes which do not appear to match the understanding of classical cellular differentiation (i.e. co-expression of CD45 [leukocyte] and cytokeratin [epithelial], CD11c/CD14 [macrophage] and CD41 [macrophage/megakaryocyte], CD146 [endothelial] and CD61 [macrophage/endothelial/megakaryocyte], CD31 [white blood cell/macrophage/endothelial/megakaryocyte/stem cell] and CD68/CD163 [macrophage]). Many of the markers appear on multiple cell types. Combined, these data show CAMLs are myeloid-derived cells early in their differentiation process that possess many phenotypic attributes associated with stem cell and proangiogenic capabilities.

CAMLs can be visualized by colorimetric stains, such as H&E, or fluorescent staining of specific markers as shown in **FIG. 9****.** For the cytoplasm, CD31 is the most positive phenotype. CD31 alone, or in combination with other positive markers in **FIG. 9****,** or cancer markers associated with the tumor are recommended.

Thus, and in the various embodiments and aspects of the invention, CAMLs can be defined as having each of the following characteristics and can be identified in a biological sample based on these characteristics alone:
(a) large atypical polyploid nucleus of about 14-64 µm in size, or multiple nuclei in the same cell;
(b) cell size of about 20-300 µm in size; and
(c) morphological shape selected from the group consisting of spindle, tadpole, round, oblong, two legs, more than two legs, thin legs, and amorphous.

In certain aspects of the embodiments of the invention, CAMLs can be further defined as having one or more of the following additional characteristics:
(d) CD14 positive phenotype;
(e) CD45 expression;
(f) EpCAM expression;
(g) vimentin expression;
(h) PD-L1 expression;
(i) CD11C marker expression;
(j) CD146 marker expression;
(k) CD202b marker expression;
(l) CD31 marker expression; and
(m) CK8, 18, 19 epithelial phenotype.

For metastatic cancer, diagnostic information obtained from a patient is useful in methods of: (i) eliminating cancer at metastatic sites and (ii) the treatment and/or cure of the cancer, i.e. elimination cancer everywhere in the subject. The reduction of CAML numbers is an indicator of tumor regression and clearance in the metastatic sites. This concept is applicable to all treatments and all cancers that are associated with large number of CAMLs. For example, in subjects having breast cancer, there is usually a large number of CAMLs, with approximately 30-50 CAMLs per 7.5 mL of blood in Stage IV patients. In contrast, in subjects having lung cancer, there is typically only about 5 CAMLs per 7.5 mL of blood in Stage IV patients. The existence of CAMLs is an indicator of presence of cancer. CAML size is also an indicator of the aggressiveness of the disease.

### Circulating Tumor Cells

CTCs express a number of cytokeratins (CKs). CK 8, 18, & 19 are the most commonly used in diagnostics, and can be used in the methods of the present invention, but surveying need not be limited to these three. The surface of solid tumor CTCs usually express epithelial cell adhesion molecule (EpCAM). However, this expression is not uniform or consistent. CTCs should not express any CD45, because it is a white blood cell marker. In assays to identify tumor associated cells, such as CTCs and CAMLs, it is sufficient to use antibody against CK 8, 18, or 19, or antibody against CD45 or DAPI. Combining the presence of staining with morphology, pathologically-definable CTCs, apoptotic CTCs and CAMLs can be identified.

A pathologically-definable CTC is identified by the following characteristics:
- They have a "cancer-like" nucleus stained by DAPI. The exception is when the cell is in division; the nucleus is condensed.
- They express at least CK 8, 18 and 19. The cytokeratins have a filamentous pattern.
- They lack CD45 expression. To avoid missing low expressing CD45 cells, long exposure is used during image acquisition.

A pathologically-definable CTC of the present invention thus includes those CTCs having one, two or three of the following characteristics: (a) cancer-like nucleus; (b) expression of one or more of cytokeratin 8, 18 and 19, and wherein the cytokeratins have filamentous pattern; and (c) CD45 negative phenotype.

An apoptotic CTC is identified by the following characteristics:
- They have a cancerous nucleus.
- They express at least CK 8, 18 and 19; the cytokeratins are not filamented, but appear fragmented in the form of spots.
- They do not express CD45.

An apoptotic CTC of the present invention thus includes those CTCs having one, two or three of the following characteristics: (a) cancer-like nucleus; (b) expression of one or more of cytokeratin 8, 18 and 19, and wherein the cytokeratin is fragmented in the form of spots; and (c) CD45 negative phenotype.

### Capture of CAMLS and CTC

As suggested above, the unique characteristics of the CAMLs and CTCs described herein make them well-suited for use in clinical methodology including methods of screening and diagnosis diseases such as cancer, monitoring treatment, monitoring of disease progression and recurrence.

Cells larger and/or less flexible than other cells present in a bodily fluid, e.g. CMALs and CTCs, may be collected by filtering the bodily fluid. For example, targeted cells indicative of a condition may be collected by passing a bodily fluid through a filter having openings that are too small for the target cells to pass through, but large enough for other cells to pass through. Once collected, any number of analyses of the target cells may be performed. Such analyses may include, for example, identifying, counting, characterizing expressions of markers, obtaining molecular analysis, and/or culturing the collected cells.

In each of the embodiments and aspects of the invention, the circulating cells (e.g., CAMLs and CTCs) may be isolated from the biological samples in the methods of the invention using any relevant means known to the skilled artisan. Suitable means include, but are not limited to, one or more means selected from size exclusion methodology, immunocapture, red blood cell lysis, white blood cell depletion, FICOLL separation, electrophoresis, dielectrophoresis, flow cytometry, magnetic levitation, and various microfluidic chips via physical size-based sorting, slits, channels, hydrodynamic size-based sorting, grouping, trapping, concentrating large cells, eliminating small cells, or a combination thereof. In a particular aspect, the size exclusion methodology comprises use of a microfilter.

As an example, circulating cells (e.g., CAMLs and CTCs) may be isolated from the biological samples using size exclusion methodology that comprises using a microfilter. Suitable microfilters can have a variety of pore sizes and shapes. For example, the microfilter may have a pore size ranging from about 5 microns to about 20 microns. Suitable ranges of pore sizes also include, but are not limited to, pore sizes ranging from about 5-7 microns, 7-8 microns, 8-10 microns, 11-13 microns, 14-16 microns, 17-20 microns, 5-10 microns, 11-15 microns, 15-20 microns, 9-15 microns, 16-20 microns, and 9-20 microns. Another suitable range comprises pore sizes ranging from about 5-20 microns, but excluding pores of between 7 and 8 microns. In certain aspects of the invention, the pore size is between about 5 and 10 microns; in other aspects, the pore size is between about 7 and 8 microns. The larger pore sizes will eliminate most of the white blood cell (WBC) contamination on the filter. The pores of the microfilter may have any shape, with acceptable shapes including round, race-track shape, oval, slit, square, rectangular and/or other shapes. The microfilter may have precision pore geometry, uniform pore distribution, more than one pore geometry, and/or non-uniform distribution. The microfilter may be single layer, or multi-layers with different shapes on different layers.

Circulating cells (e.g., CAMLs and CTCs) may also be isolated from the biological samples using a microfluidic chip via physical size-based sorting, slits, channels, hydrodynamic size-based sorting, grouping, trapping, immunocapture, concentrating large cells, or eliminating small cells based on size. The circulating cell capture efficiency can vary depending on the collection method. The circulating cell size that can be captured on different platforms can also vary. The principle of using circulating cell size to determine disease progression and treatment response is the same, but the statistics will vary. Collection of circulating cells using CellSieve^{™} microfilters provides 100% capture efficiency and high quality cells.

Biological samples, especially when the sample is blood, may be collected in blood collection tubes. CellSave blood collection tubes (Menarini Silicon Biosystems Inc., San Diego, CA) provide stable cell morphology and size. Other available blood collection tubes may not provide cell stability. Cells can enlarge and may even burst in most other blood collection tubes.

The size of the biological samples assayed in the methods of the invention can vary without changing the underlying theory and characteristics on the invention. Suitable sample sizes generally range from about 0.5 mL to about 50 mL. Suitable sizes include samples ranging from about 5 to about 15 mL, from about 5 to about 10 mL, from about 10 to about 15 mL, from about 15 to about 20 mL, from about 20 to about 25 mL, from about 25 to about 30 mL, from about 30 to about 35 mL, from about 35 to about 40 mL, from about 40 to about 45 mL, and from about 45 to about 50 mL. In certain aspects of the invention, the size of the biological sample is about 7.5 mL.

The source of the biological samples used in the methods of the invention is only limited in that it must contain one of the types of circulating cells mentioned herein, e.g. CAMLs and/or CTCs. Suitable sources for the biological samples include blood, lymph node, bone marrow, cerebral spinal fluid, tissue, urine, peripheral blood mononuclear cells (PBMCs), and cryopreserved PBMCs. When the biological sample is blood, the blood may be peripheral blood, antecubital-vein blood, inferior-vena-cava blood, femoral vein blood, portal vein blood, or jugular-vein blood, for example. The sample may be a fresh sample or a properly prepared cryo-preserved sample that is thawed.

In a further aspect of the invention, circulating cells (CAMLs) are isolated from the biological samples using a CellSieve^{™} low-pressure microfiltration assay.

When comparing circulating cell sizes between two subjects having cancer, it is preferable for the subjects to have the same type of cancer. However, it may be difficult to fully match two subjects in terms of the type of cancer, the stage of the cancer, the rate of progression of the cancer, history of treatment, and history of remission and/or reoccurrence of the cancer, among other factors. Therefore, it should be understood that there may be some variations in the characteristics in the cancers of two subject that are being compared in the relevant methods of the invention.

### Identification of CAMLs

As indicated above, the various embodiments and aspects of the invention are based on determining the size or number of CMALs in a sample obtained from a subject having cancer. Specific means for identifying CAMLs in a sample are mentioned above and include: (i) determining the size, shape and number of nuclei in the cell; (ii) determining overall cell size; (iii) determining morphological shape of the cell; and (iv) use of one or more of the markers shown in Figure 9.

However, it should be made clear that each of the embodiments and aspects of the invention may be practiced without definitively identifying circulating cells in the biological samples as CAMLs *per se.* Instead, simply identifying the cells based on size of the cell, for example may be used. Examples of other techniques includes using color-metric stains, such as H&E stains, or simply identifying CK (+) cells in or collected from a biological sample.

### Subjects

The subjects mentioned in the methods of the present invention will be a human, a non-human primate, bird, horse, cow, goat, sheep, a companion animal, such as a dog, cat or rodent, or other mammal. Animals also can develop cancer. Cancer causes almost 50% of deaths in pets over the age of 10. Some common types of cancers in pets include: skin, breast, head and neck, lymphoma, leukemia, testicular, abdominal, and bone. Examples of cancers commonly found in pets that are also commonly found in humans are lymphoma, melanoma, and osteosarcoma. CAMLs are found in non-human animals. Therefore the clinical utility of CAMLs for humans is also applicable to other animals.

In the various aspects and embodiments of the invention, the subject is a subject having cancer. The cancer may be a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer. The skilled artisan will appreciate that the methods of the invention are not limited to particular forms or types of cancer and that they may be practiced in association with a wide variety of cancers.

### Treatment

In certain aspects and embodiments of the invention, the subject is undergoing treatment. The treatment may be one or more of chemotherapy, single drug, combination of drugs, immunotherapy, radiation therapy, chemoradiation, radiation combined with single or multiple drug, chemoradiation combined with single or multiple drugs, cancer vaccine, and cell therapy.

Cancer vaccines can be administered to a subject in the form of cells expressing the markers intended as vaccine targets. The cells can be in the form of cancer cells, modified viruses and other types of modified cells. After the subject receives the vaccine, the body's immune system produces T cells that recognize and attack antigens expressed by the vaccine as well as cancer cells. When the treatment is a cancer vaccine, the subject may express at least one HLA allele.

CAML size is still predictive of the aggressiveness of the cancer. Additional information can be obtained by CAML number. The immune system may be able to eliminate cancer from smaller metastatic sites faster than the largest cancer sites. The killer T cells of the immune system may be more able to penetrate tumors in some organs than others. For example, immunotherapy typically works the best for lung cancer and melanoma, but less well for breast and prostate cancers. Often, as the tumor becomes larger, pseudo progression is seen, as immune cells enter the tumor to kill the tumor cells. Pseudo progression in an indicator of good immunotherapy treatment response.

An example of cancer vaccine is SV-BR-1-GM, a GM-CSF-engineered breast cancer cell line expressing HER2, FRAME and Class I and Class II HLA antigens. SV-BR-1-GM cells loaded with a yellow fever virus (YFV) peptide directly activated YFV-specific CD4+ T cells.

The responders of this SV-BR-1-GM vaccine is predicted to be breast cancer patients that express at least one HLA allele.

### Methods of the Invention

As described in the Summary above, the present invention is directed, *inter alia,* to methods of predicting treatment response and or disease progression (e.g., progression of cancer) in a subject based on circulating cell numbers and/or size. These methods comprise determining the numbers and/or size of circulating cells in a biological sample from a subject, such as a subject having cancer, and in some aspects and embodiments, comparing the results from samples taken from the same subject at a different time point. The change of the size or the number of the circulating cells being assayed is an indicator of treatment response and/or disease progression in the subject and predictions can be based thereon.

Specific embodiments and aspects of the invention are presented in the paragraphs below. However, it can first be noted here that when the methods of the invention are associated with predicting treatment responses in a subject, the time scale to obtain treatment response information can generally be as short as two treatment cycles or 60 days, depending on the type of treatment. It will be apparent that treatment response may also be seen after one treatment cycle, or in as little as 10 days, depending on the type of treatment.

It can also be noted here that CAML size and number, and changes in CAML size and number, can be analyzed together with other parameters to improve the probability of correct determination of treatment response and/or disease progression. Such parameters include:
1. Good treatment response, e.g.:
   - disappearance of CTCs
   - decrease in CTC number when they are very high initially
   - decrease in circulating tumor DNA (ctDNA)
2. Poor treatment response, e.g.:
   - increase in CTC number [3]
   - appearance of CTCs in mitosis [7]
   - appearance of CTCs in clusters
   - increase in the amount of ctDNA
   - increase in mutation in ctDNA.
   - increase in mutation in CAML DNA.

Further, CAML size and number, and changes in CAML size and numbe, can provide prediction of treatment response and/or disease progression before imaging may be able to provide prognostic information. Imaging requires the tumor to grow substantially to see the difference in disease progression. CT imaging also produces undesirable radiation dose, which can cause cancer and it is not desirable at 30-45 day intervals. Finally, imaging is expensive.

An increase in the number of CTCs is known to form the basis for predictions on disease progression. However, it is only applicable by itself for Stage IV breast, prostate and colorectal cancers. CTCs are rare in earlier stages of cancer and other types of solid tumors.

### Determining Circulating Cell Size

In one embodiment, the methods of the invention comprise determining the size of circulating cells, such as CAMLs, in two or more biological samples obtained from a subject having cancer over time, wherein when a decrease in size of the circulating cells between the samples is found over time, cancer is predicted not to progress and/or the subject is found to be responding to treatment. In related embodiment, the methods of the invention comprise determining the number of circulating cells, such as CAMLs, in two or more biological samples obtained from a subject having cancer over time, wherein when a decrease in the number of the circulating cells between the samples is found over time, cancer is predicted not to progress and/or the subject is found to be responding to treatment.

In a related aspect, the method comprises determining the size of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the size of at least one cell in the first sample is about 50 µm or more, and the size of each cell in the second and optional additional samples is less than about 50 µm, cancer is predicted not to progress and/or the subject is found to be responding to treatment.

In another related aspect, the method comprises determining the average size of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the average size of circulating cells in the second and optional additional samples is decreased in comparison to the average size of the cells in the first sample, the subject is identified as responding to treatment. In some aspects, the average size of the circulating cells in the first sample is about 50 µm or more.

### Predicting Cancer Progression

In another embodiment, the invention is directed to methods for predicting cancer progression in a subject having cancer comprising determining the size of circulating cells, such as CAMLs, in a biological sample obtained from a subject having cancer, and making a prediction based thereon, wherein when each circulating cell in the sample is less than about 50 µm in size, the cancer is predicted not to progress, and wherein when at least one circulating cell in the sample is about 50 µm or more in size, the cancer is predicted to progress.

In one aspect of the embodiment, the invention is directed to methods for predicting cancer progression in a subject having a cancer comprising determining the size of circulating cells, such as CAMLs, in a first and second biological sample, and optional additional biological samples, obtained from a subject having cancer, wherein the first sample is obtained from the subject prior to or during cancer treatment, wherein the second sample and optional additional samples are obtained from the subject after at least one cancer treatment,
wherein when the average size of CAMLs in the second and optional additional samples is decreased in comparison to the average size of the CAMLs in the first sample, the cancer is predicted not to progress in the subject and the subject is optionally identified as responding to treatment; or
wherein when the average size of CAMLs in the second and optional additional samples is maintained or increased in comparison to the average size of the CAMLs in the first sample, the cancer is predicted to progress in the subject and the subject is optionally identified as not responding to treatment; or
wherein when the size of at least one CAML in the first sample is about 50 µm or more, and the size of each cell in the second and optional additional samples is less than about 50 µm, the cancer is predicted not to progress and the subject is optionally identified as responding to treatment; or
wherein when the size of each CAML in the first sample is less than about 50 µm, and when at least one CAML in the second and optional additional samples is greater than about 50 µm in size, the cancer is predicted to progress and the subject is optionally identified as not responding to treatment. In some aspects, the average size of the circulating cells in the first sample is about 50 µm or more.

In another aspect of the embodiment, the invention is directed to methods for predicting cancer progression in a subject having a cancer comprising determining the number of circulating cells, such as CAMLs, in a first and second biological sample, and optional additional biological samples, obtained from a subject having cancer, wherein the first sample is obtained from the subject prior to or during cancer treatment, wherein the second sample and optional additional samples are obtained from the subject after at least one cancer treatment, wherein when the number of circulating cells in the second and optional additional samples is decreased in comparison to the number of the circulating cells in the first sample, the cancer is predicted not to progress and the subject is optionally identified as responding to treatment, and wherein when the number of circulating cells in the second and optional additional samples is maintained or increased in comparison to the number of the circulating cells in the first sample, the cancer is predicted to progress and the subject is optionally identified as not responding to treatment.

### Predicting Treatment Response

In a further embodiment, the invention is directed to methods for predicting response to treatment of a subject having cancer comprising determining the size of circulating cells, such as CAMLs, in a biological sample obtained from the subject, and making a prediction based thereon, wherein when each circulating cell in the sample is less than about 50 µm in size, the subject is predicted to respond to treatment, and wherein when at least one circulating cell in the sample is about 50 µm or more in size, the subject is predicted to not respond to treatment.

In one aspect of the embodiment, the invention is directed to methods for predicting response to treatment of a subject having cancer comprising determining the size of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, and
wherein when the average size of CAMLs in the second and optional additional samples is decreased in comparison to the average size of the CAMLs in the first sample, the subject is predicted to respond to treatment;
wherein when the average size of CAMLs in the second and optional additional samples is maintained or increased in comparison to the average size of the CAMLs in the first sample, the subject is predicted to not respond to treatment;
wherein when the number of circulating cells greater than about 50 µm in size is maintained or increases from the first to the second and optional additional samples, the subject is predicted to not respond to treatment; or
wherein when the number of circulating cells greater than about 50 µm in size decreases from the first to the second and optional additional samples, the subject is predicted to respond to treatment; or
wherein when the size of each circulating cell in the second or following sample decreases in comparison to the first sample, and wherein the size of each circulating cell in the second or optional additional sample is less than about 50 µm, the subject is predicted to respond to treatment and potentially cured of cancer.

In another aspect of the embodiment, the invention is directed to methods for predicting response to treatment of a subject having cancer comprising determining the absence of any circulating cells, such as CAMLs, larger than about 50 µm in a biological sample from the subject and predicting the subject is responding to treatment.

In a further aspect of the embodiment, the invention is directed to methods for predicting response to treatment of a subject having cancer comprising determining the size of circulating cells, such as CAMLs, in a biological sample obtained from a subject having cancer, wherein the sample is obtained from the subject after at least one cancer treatment, and wherein when each circulating cell is about 50 µm or less in size, the subject is predicted to respond to treatment.

In an additional aspect of the embodiment, the invention is directed to methods for predicting response to treatment of a subject having cancer comprising determining the number of circulating cells, such as CAMLs, in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the number of circulating cells decreases from the first to the second and optional additional samples, the subject is predicted to respond to treatment, and wherein when the number of circulating cells is maintained or increases from the first to the second and optional additional samples, the subject is predicted to not respond to treatment.

### Predicting Resistance to Treatment

Disclosed but not claimed are methods predicting resistance to treatment in a subject having lung cancer, said method comprising determining the size of circulating cells, such as CAMLs, in a biological sample from a subject having cancer, wherein when at least one cell in said sample is about 50 µm or more in size, the subject is predicted to be more resistant to cancer treatment than a subject having cancer where none of the cells is more than about 50 µm in size. In certain aspects the cancer is lung cancer.

If no circulating cells are found in the biological sample at the end of the treatment, it may be concluded that the cancer has been eliminated. When circulating cells are still found in the biological sample at the end of the treatment, it may be concluded that the cancer in the patient has not been eliminated, and that the patient has residual disease.

CAMLS may be used independently as a cancer marker, or in combination with other circulating cells, such as circulating tumor cells (CTCs), epithelial mesenchymal transition calls (EMTs), and circulating cancer associated vascular endothelial cells (CAVEs), as well as with cell-free DNA (cfDNA), circulating tumor DNA (ctDNA), methylated DNA, proteomic, metabolomic, lipidomic and other biomarkers to provide a more complete understanding of a patient's disease. CAMLs are the only cell type among the group of circulating cells mentioned herein that is typically larger than 30 um in size.

### Methods of Treatment

Disclosed but not claimed are methods of treating cancer, where treatment decisions can be based on predictions of treatment response using the circulating cells in the methods described above.

The disclosure thus encompasses methods of treating a subject having cancer comprising administering a therapeutically effective amount of a cancer treatment to a subject having cancer and determining the size of circulating cells, such as CAMLs, in two or more biological samples obtained from a subject having cancer, wherein a first biological sample is obtained from the subject before or during the cancer treatment, wherein a second biological sample is obtained from the subject during or after the cancer treatment, wherein when a decrease in size of the circulating cells between the samples is found over time, the subject is found to be responding to treatment and the treatment is continued, and wherein when a decrease in size of the circulating cells between the samples is not found over time, the subject is found not to be responding to treatment and the treatment is not continued.

Related methods comprise administering a therapeutically effective amount of a cancer treatment to a subject having cancer and determining the number of circulating cells, such as CAMLs, in two or more biological samples obtained from a subject having cancer, wherein a first biological sample is obtained from the subject before or during the cancer treatment, wherein a second biological sample is obtained from the subject during or after the cancer treatment, wherein when a decrease in the number of the circulating cells between the samples is found over time, the subject is found to be responding to treatment and the treatment is continued, and wherein when a decrease in the number of the circulating cells between the samples is not found over time, the subject is found not to be responding to treatment and the treatment is not continued.

In an aspect, the method comprises administering a therapeutically effective amount of a cancer treatment to a subject having cancer and determining the size of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, wherein when the size of at least one cell in the first sample is about 50 µm or more, and the size of each cell in the second and optional additional samples is less than about 50 µm, the subject is found to be responding to treatment and the treatment is continued.

In another related aspect, the method comprises administering a therapeutically effective amount of a cancer treatment to a subject having cancer and determining the average size of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, wherein when the average size of circulating cells in the second and optional additional samples is decreased in comparison to the average size of the cells in the first sample, the subject is identified as responding to treatment and the treatment is continued, and wherein when the average size of circulating cells in the second and optional additional samples is not decreased in comparison to the average size of the cells in the first sample, the subject is identified as not responding to treatment and the treatment is not continued. In some aspects, the average size of the circulating cells in the first sample is about 50 µm or more.

Disclosed but not claimed are methods of treating a subject having cancer comprising administering a therapeutically effective amount of a cancer treatment to a subject having cancer and determining the size of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, and
wherein when the average size of CAMLs in the second and optional additional samples is decreased in comparison to the average size of the CAMLs in the first sample, the subject is predicted to respond to treatment and the treatment is continued;
wherein when the average size of CAMLs in the second and optional additional samples is maintained or increased in comparison to the average size of the CAMLs in the first sample, the subject is predicted to not respond to treatment and the treatment is not continued;
wherein when the number of circulating cells greater than about 50 µm in size is maintained or increases from the first to the second and optional additional samples, the subject is predicted to not respond to treatment and the treatment is not continued;
wherein when the number of circulating cells greater than about 50 µm in size decreases from the first to the second and optional additional samples, the subject is predicted to respond to treatment and the treatment is continued; or
wherein when the size of each circulating cell in the second or following sample decreases in comparison to the first sample, and wherein the size of each circulating cell in the second or optional additional sample is less than about 50 µm, the subject is predicted to respond to treatment and the treatment is continued.

Disclosed but not claimed are methods of treating a subject having cancer comprising administering a therapeutically effective amount of a cancer treatment to a subject having cancer and determining the absence of any circulating cells, such as CAMLs, larger than about 50 µm in a biological sample obtained from the subject, wherein the sample is obtained from the subject after at least one cancer treatment, and wherein when there is an absence of any circulating cells larger than about 50 µm in the biological sample the subject is predicted to respond to treatment and the treatment is continued.

Disclosed but not claimed are methods of treating a subject having cancer comprising administering a therapeutically effective amount of a cancer treatment to a subject having cancer and determining the size of circulating cells, such as CAMLs, in a biological sample obtained from the subject, wherein the sample is obtained from the subject after at least one cancer treatment, and wherein when each circulating cell is about 50 µm or less in size, the subject is predicted to respond to treatment and the treatment is continued.

Disclosed but not claimed are methods of treating a subject having cancer comprising administering a therapeutically effective amount of a cancer treatment to a subject having cancer and determining the number of circulating cells, such as CAMLs, in a first and second biological sample, and optional additional biological samples, obtained from the subject, wherein the first sample is obtained from the subject prior to or during cancer treatment, wherein the second and optional additional samples are obtained from the subject after at least one cancer treatment, wherein when the number of circulating cells decreases from the first to the second and optional additional samples, the subject is predicted to respond to treatment and the treatment is continued, and wherein when the number of circulating cells is maintained or increases from the first to the second and optional additional samples, the subject is predicted to not respond to treatment and the treatment is not continued.

### EXAMPLES

### Example 1

### Sample collection and processing

Size exclusion is one method to collect both CTCs and CAMLs. There are many size exclusion methods. Filtration method is given here as an example. Peripheral blood was collected in Cell Save Tubes (Menarini Silicon Biosystems Inc., San Diego, CA) and processed within 96 hours. CellSieve^{™} microfiltration technique was used to collect all cancer associated cells (CTCs, EMTs, CECs, and CAMLs) in the blood sample. CellSieve^{™} microfilters have greater than 180,000 pores in uniform array with 7 µm pore diameter within a 9 mm area. The reagents include prefixation buffer, postfixation buffer, permeabilization buffer, and an antibody cocktail. The technique to perform the filtration used either a syringe pump set drawn at 5 mL/min [5] or a vacuum pump [4]. The filtration process started by prefixing 7.5 mL of the blood in 7.5 mL of prefixation buffer before drawn through the filter. The filter and captured cells were then subjected to washing, postfixation, washing, permeabilization and washing. Then, the captured cells on the filter were stained with an antibody cocktail followed by wash. Filters were placed onto a microscope slide and cover-slipped with Fluoromount-G/DAPI (Southern Biotech). One possible antibody cocktail to identify CTCs and CAMLs in epithelial cancers can contain cytokeratins and CD45 antibodies. Additional antibodies for markers of interest can be included. The slide is then read on a fluorescent microscope. Number of CTCs are counted. Size of CAMLs are measured. The number of CTCs, the largest CAML size, and the largest CAML size < 50 µm or ≥ 50 µm are recorded. There are a wide variety of other methods to collect CTCs and CAMLs from blood.

### Study 1: Study of n=42 NSCLC Patients Treated by Radiation

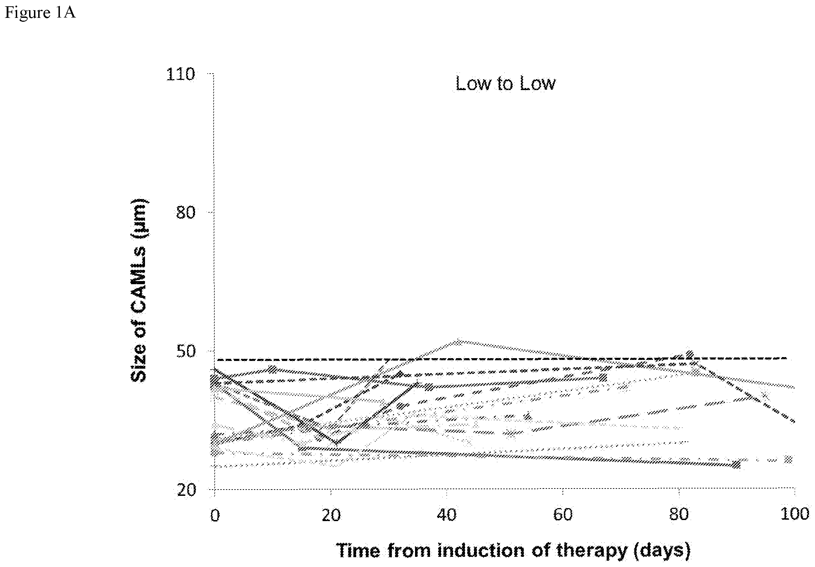

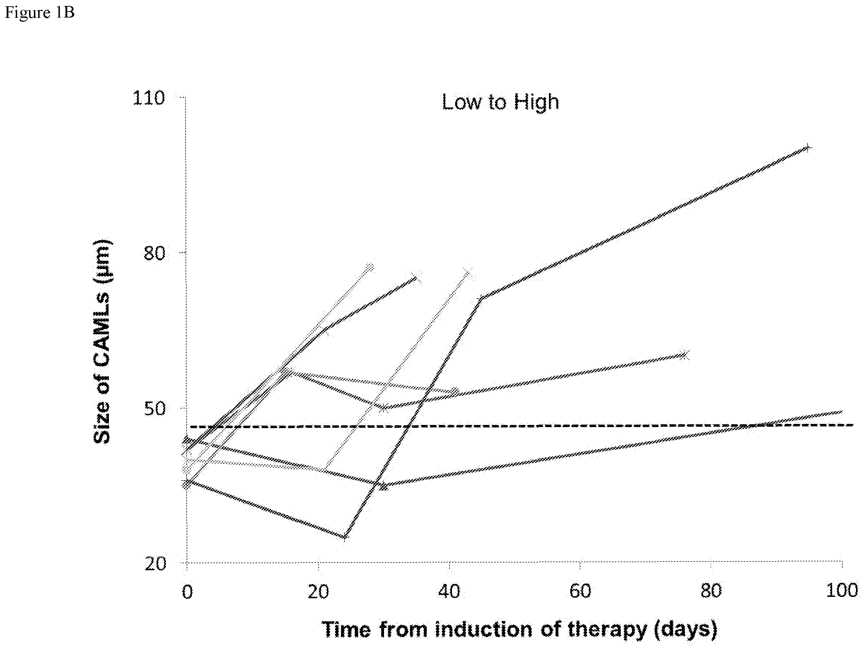

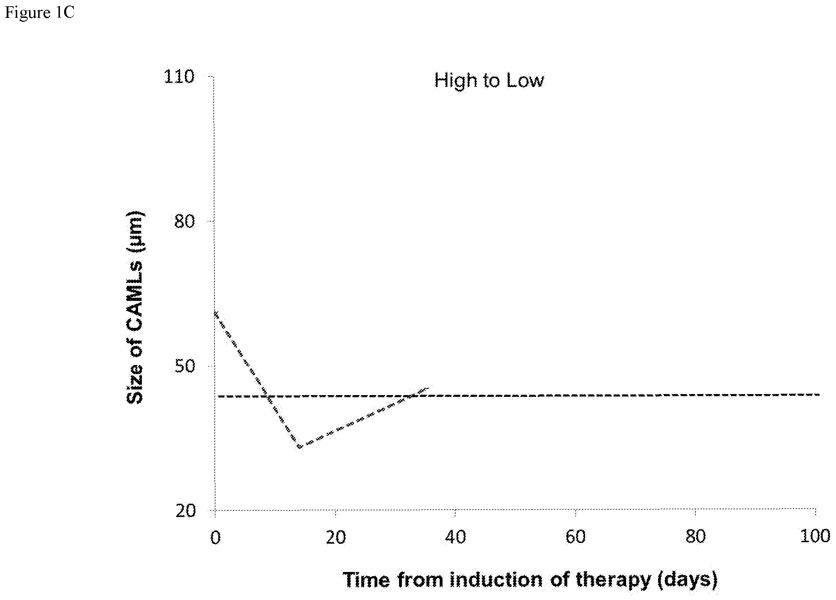

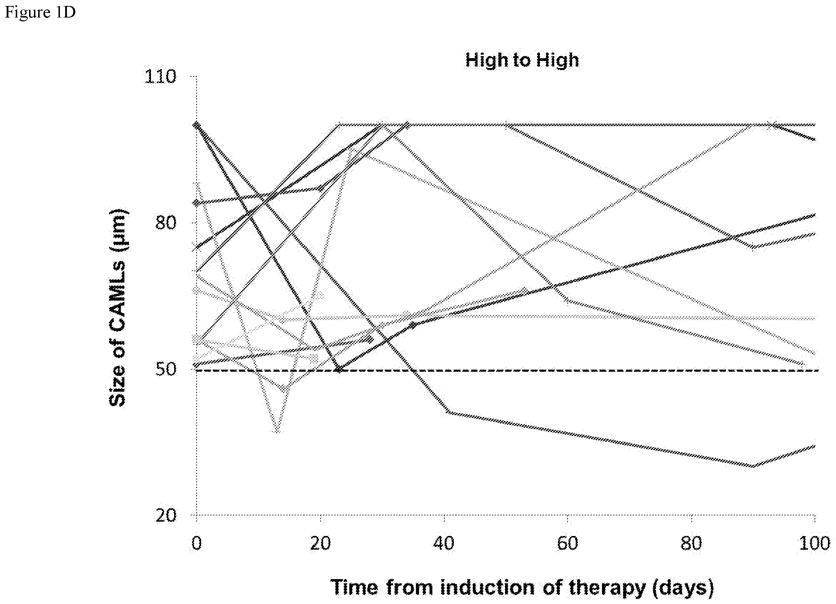

**FIGs 1A-1D** show the CAML size and changes of CAML size related to treatment response. The solid curves and lines indicate non-responders, while the dashed indicate responders. For data analysis of this figure, the treatment response was defined as response at a 24 month time-point. For this pilot study of n=42 sample, the subjects had non-small cell lung cancer (NSCLC) and most of them were undergoing radiotherapy and a few were also receiving chemotherapy. Progression is based on standard of care imaging in lung patients before and after induction of chemoradiotherapy. The results even at the first 100 days show a strong trend. **FIG. 1A** shows that most of the patients with CAMLs smaller than 50 µm in size at baseline remained less than 50 µm. **FIG. 1B** shows all of the patients with CAMLs increasing in size from less than 50 µm to larger than 50 µm progressed. **FIG. 1C** shows that one patient initially having CAMLs larger than 50 µm and at second time point having CAMLs smaller than 50 µm did respond to therapy. **FIG. 1D** shows no response to treatment in patients where CAMLs were larger than 50 µm at baseline and there was no reduction in size over time, as well for one subject that did exhibit CAML size reduction.

To provide a qualitative measurement, accuracy of prediction at a given time point was defined as (true positives + true negatives)/(false positives + false negatives). True positives are number of patients with CAML size >50 µm and progressed. False positives are number of patients with CAML size >50 µm and but did not progress. True negatives are number of patients with CAML size <50 µm and did not progress. False negatives are number of patients with CAML size <50 µm and progressed. This use of 50 µm size is based on data shown in international patent application publication WO 2018/151865, dated August 23, 2018. The accuracy at baseline was 69%, and the accuracy at last follow up was 85%.

The indication of treatment response can be obtained within 100 days for most patients. In summary, the final CAMLs size after treatment provides the basis for making predictions and conclusions regarding treatment response.

### Study 2: Study of n=52 NSCLC Patients Treated by Radiotherapy

To achieve a 2-tailed 90% power with an α of 0.05, a test set of 52 NSCLC patients was expended. All patients had pathologically confirmed lung cancers Stage I (n=7), Stage II (n=7), Stage III (n=29) & Stage IV (n=9), and received standard PET/CT scans. Baseline (BL) blood sample was taken prior to start of therapy. The first follow up (FU1) were taken during radiotherapy (~30 days). The second follow up (FU2) were taken at the end of radiotherapy (~60 days). A few patients also received chemotherapy within the 60 days.

Blood was filtered by CellSieve^{™} microfiltration and CAML sizes were quantified. Analysis by CAML size of <50 µm or ≥50 µm was used to evaluate PFS hazard ratios (HRs) by censored univariate & multivariate analysis at each time point.

The analysis below predicts disease progression, opposite of treatment response. CAMLs were identified in 97% of all samples averaging 2.9 CAMLs/7.5mL sample at BL, with CAMLs of ≥50 µm having reduced progression free survival (PFS) (HR=2.9, 95%CI1.3-6.2, p=0.015). At FU1 (follow up 1), 7 patients had an increased CAML size which reduced PFS (HR=5.0 95%CI2.3-10.9, p<0.001). At FU2 (follow up 2), 7 additional patients had increased CAML size further reducing PFS (HR=7.1, 95%CI3.4-14.8, p<0.001). In total, CAMLs with size ≥50 µm at BL was 65% accurate at predicting eventual progression within 24 months while CAMLs with size ≥50 µm at FU2 was 89% accurate at predicting progression.

Note that is applicable to predicting treatment response or progression in all stages of NSCLC.

### Study 3: n=52 NSCLC and n=29 Esophageal Cancer Patients Treated by Chemoradiation

A larger study was conducted of predicting radiation therapy results including esophageal cancer based on the same 52 NSCLC as described in FIGS. 1A-1D (stage I, n=7, stage II, n=7, stage III, n=29, and stage IV, n=9) and 21 esophageal cancer (stage III n=20 and Stage IV (n=1) patients.

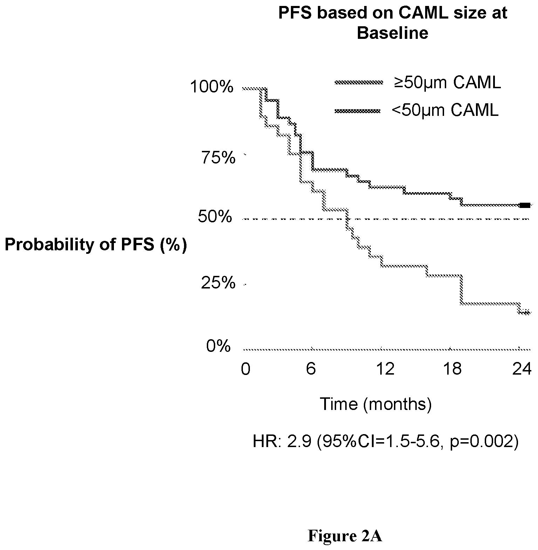

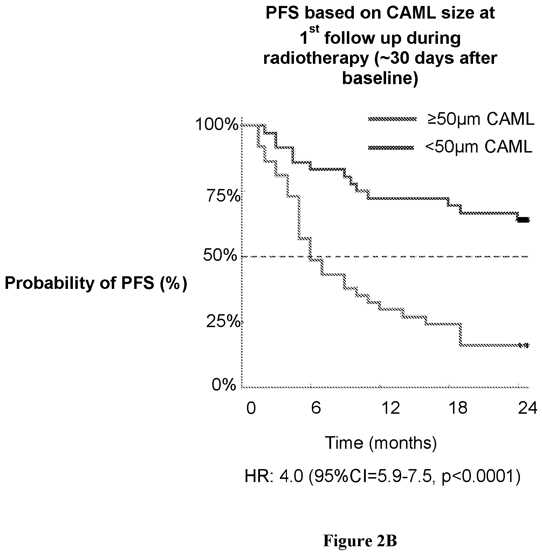

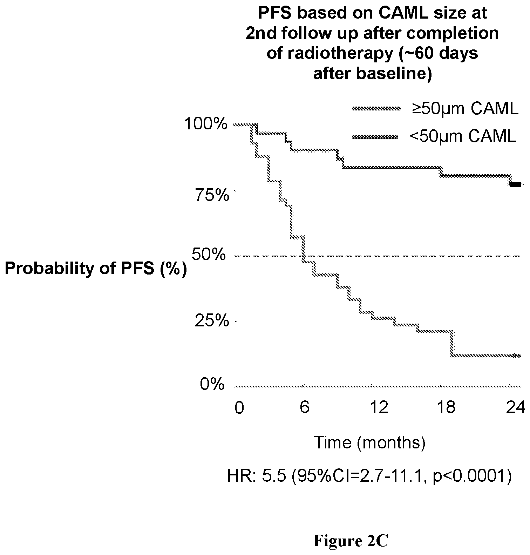

**Figures 2A-2C** show the Kaplan Meier plots of progression free survival (PFS) at three time points: baseline (BL) **(****FIG. 2A****),** first follow up (FU1) **(****FIG. 2B****),** and second follow up (FU2) **(****FIG. 2C****).** The data is analyzed based on the size of the CAMLs, < 50 µm (blue (upper) curves) or ≥ 50 µm (red (lower) curves). At baseline **FIG. 2A** and **Table 1,** the sizes of the CAMLs provide prognosis information. At first follow up **FIG. 2B** and **Table 2,** there is indication of predicting treatment response based on CAML size. At the second follow up after ending radiotherapy **FIG. 2C** and **Table 3,** CAML size provided much better prediction of PFS of responders from non-responders. A multi-variant analysis was performed. The CAML size provided the best results compared to stage, type of cancer, age and sex.

**Table 1**

| **5 Variable** | **PFS (p value)** | **2 Variable** | **PFS (p value)** |
|---|---|---|---|
| **Stage** | 0.014 | **Stage** | 0.005 |
| **CAML Size BL (<50 µm vs ≥50 µm)** | **0.002** | **CAML Size BL (<50 µm vs ≥50 µm)** | **0.003** |
| Esophageal/Lung | 0.587 | | |
| M/F | 0.975 | | |
| Age | 0.225 | | |

**Table 2**

| **5 Variable** | **PFS (p value)** | **2 Variable** | **PFS (p value)** |
|---|---|---|---|
| **Stage** | 0.022 | **Stage** | 0.014 |
| **CAML Size FU1 (<50 µm vs ≥50 µm)** | **0.000** | **CAML Size FU1 (<50 µm vs ≥50 µm)** | **0.000** |
| Esophageal/Lung | 0.565 | | |
| M/F | 0.499 | | |
| Age | 0.858 | | |

**Table 3**

| **5 Variable** | **PFS (p value)** | **2 Variable** | **PFS (p value)** |
|---|---|---|---|
| **Stage** | 0.041 | **Stage** | 0.033 |
| **CAML Size FU2 (<50 µm vs ≥50 µm)** | **0.000** | **CAML Size FU2 (<50 µm vs ≥50 µm)** | **0.000** |
| Esophageal/Lung | 0.529 | | |
| M/F | 0.340 | | |
| Age | 0.748 | | |

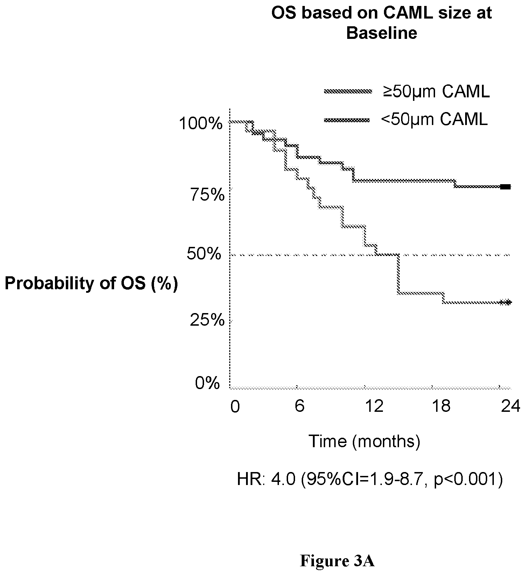

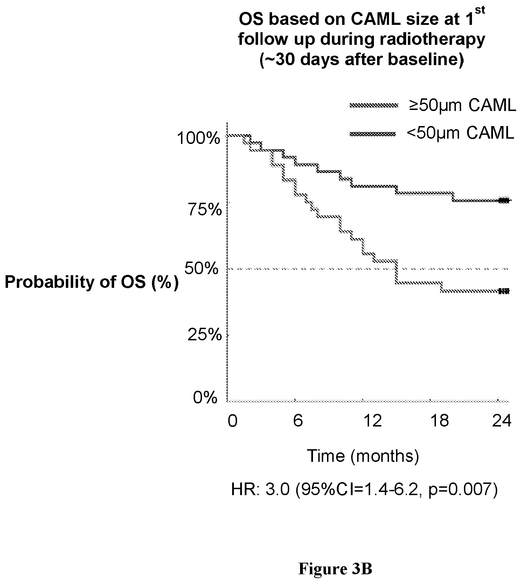

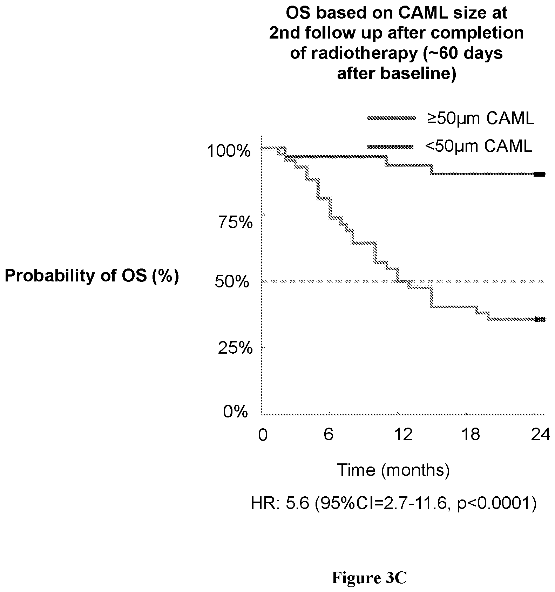

**Figures 3A-3C** show the Kaplan Meier plots of overall survival (OS) at three time points: baseline (BL) **(****FIG. 3A****),** first follow up (FU1) **(****FIG. 3B****),** and second follow up (FU2) **(****FIG. 3C****).** The data is analyzed based on the size of the CAMLs, < 50 µm (blue (upper) curves) or ≥ 50 µm (red (lower) curves). CAML size provides good prediction of OS of responders from non-responders for chemoradiation of NSCLC and esophageal cancers.

CAMLs were found in 97% of all BL samples. On average 2.9 CAMLs/7.5 mL of blood were found. Patients with giant CAMLs at BL had significantly reduced PFS for NSCLC (HR=2.9, 95%CI 1.3-6.2, p=0.015) and marginally for esophageal cancer (HR=3.0, 95%CI 0.9-9.9, p=0.14).

At FU1, patients with detectable giant CAMLs further reduced PFS after treatment (NSCLC, HR=5.0, 95%CI 2.3-10.9, p<0.001; EC, HR=4.0, 95%CI 1.2-13.2, p=0.05), and even more significantly at FU2 (NSCLC, HR=7.1, 95%CI 3.4-14.8, p<0.001; EC, HR=5.6, 95%CI 1.6-18.8, p=0.01). Combining the two disease data sets, giant CAMLs at BL was 70% accurate at predicting eventual progression within 24 months while giant CAMLs at FU2 was 84% accurate at predicting progression. On multivariable analysis, giant CAMLs was the most significant in predicting treatment response.

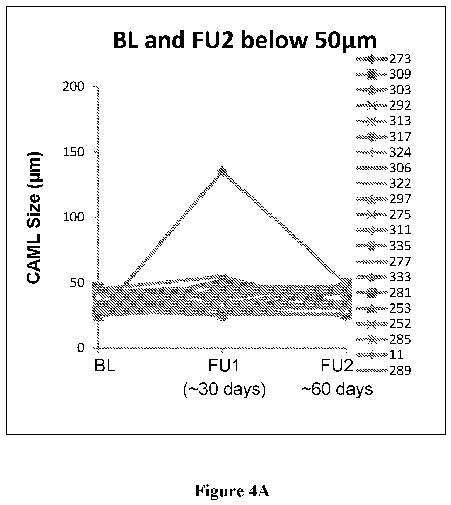

**Figures 4A-4F** show the details of largest CAML in each patient over the 3 time points, for patients having breast, prostate or lung cancer. The red and green curves are patients progressed (red) versus not progressed or cured (green).

**Figure 4A** shows the patients with CAML size below 50 µm at baseline and remained below 50 µm at FU2. 81% of the patients did NOT progress within 24 months.

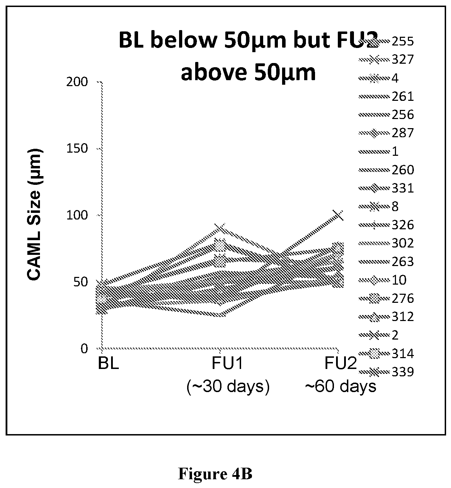

**Figure 4B** shows the patients with CAML size below 50 µm at baseline and but increased above 50 µm at FU2. 79% of the patients DID progress within 24 months.

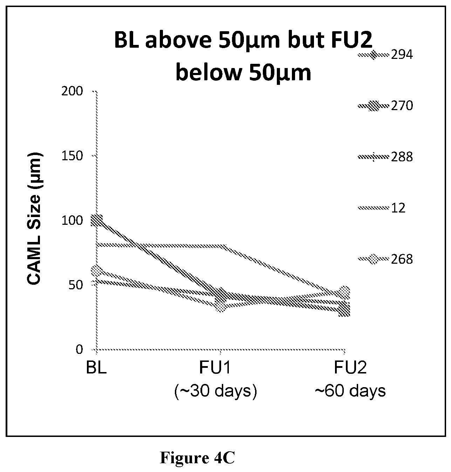

**Figure 4C** shows the patients with CAML size above 50 µm at baseline and decrease to below 50 µm at FU2. 60% of the patients did NOT progress within 24 months.

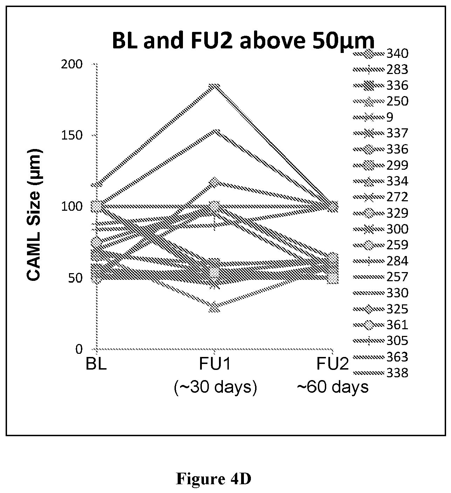

**Figure 4D** shows the patients with CAML size above 50 µm at baseline and remained above 50 µm at FU2. 96% of the patients DID progress within 24 months.

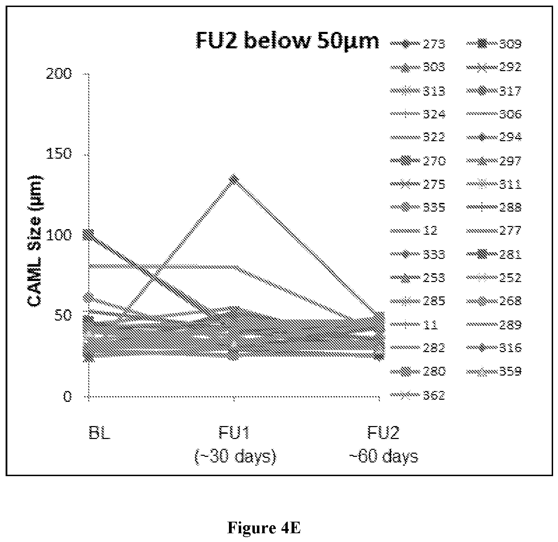

**Figure 4E** show the patients with CAML size below 50 µm at FU2. 79% of the patients did NOT progress within 24 months.

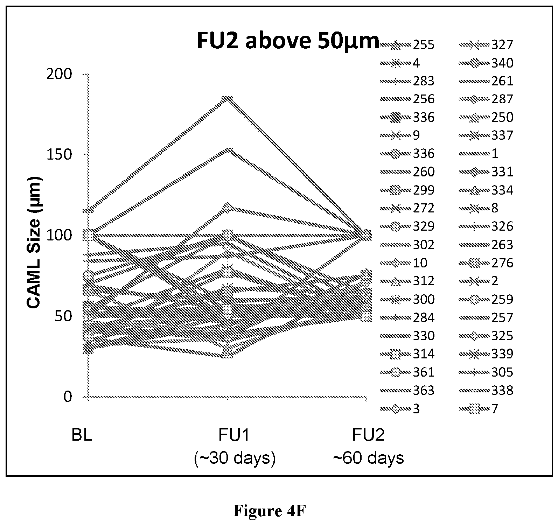

**Figure 4F** show the patients with CAML size above 50 µm at FU2. 89% of the patients DID progress within 24 months.

### Study 4: Applicable to Many Solid Tumors and Treatments

Blood based biomarkers (PSA, CEA, CA125) are used to track real time progression of disease in parallel with imaging. However while numerous blood biomarkers exist, they are specific to cancer type (i.e. PSA to prostate and CEA to colon) and may not appear in all diseased individuals. CAMLs were identified in various solid cancer types which were observed increasing in size and in hyperploidy during progressive disease. To assess whether CAML enlargement is a biomarker of progression/response, CAML growth/shrinkage was tracked in a prospective multi-institutional study using anonymized peripheral blood samples from 34 cancer patients therapy [stage I (n=2), II (n=3), III (n=8) & IV (n=21)] with breast (n=10), lung (n=16), & prostate (n=8). Samples were taken prior to therapy (BL), at ~1 month (FU1) follow up and a ~3 month (FU2) follow up, after induction of therapy.

The therapy of the patient in this group was very diverse. Therapies included: radiation, chemoradiation, chemotherapy, docetaxel, trastuzumab, trastuzumab/lapatinib, fulvestrant/trastuzumab/lapatinib/brain radiation, letrozole/denosumab,

Trastuzumab/Pertuzumab/Eribulin, paclitaxel/herceptin/pertuzumab, Abraxane, Eligard, Lupron, Eligard/Bicalutamide, Casodex, Lupron/Bicalutamide, Lupron, surgery, Abiraterone/Lupron, Nivolumab, Carboplatin/Taxol, Carboplatin/Gemcitabine, Gemcitabine/trastuzumab+lapatinib, Eribulin/trastuzumab/lapatinib, Herceptin/Fulvestrant/Palbocilib, eribulin, paclitaxel/herceptin/pertuzumab, Abraxane, Eligard+Enzalutamide, Lupron+Enzalutamide, Casodex+Lupron, Vinorelbine/trastuzumab/lapatinib, letrozole/fulvestrant, eribulin/Herceptin, herceptin/femara.

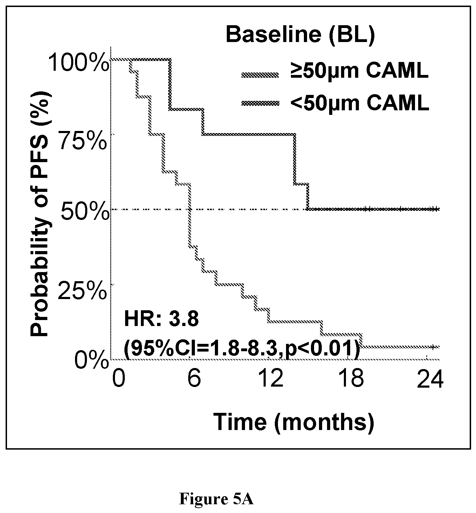

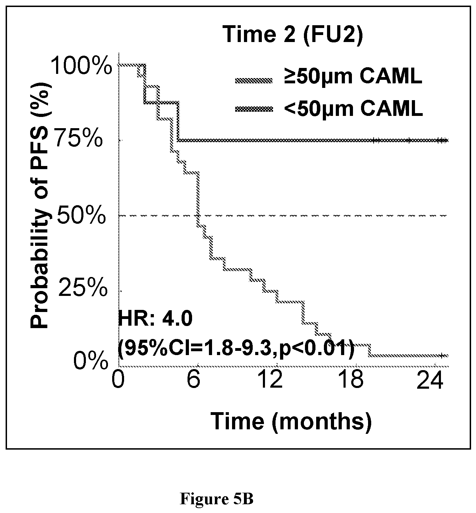

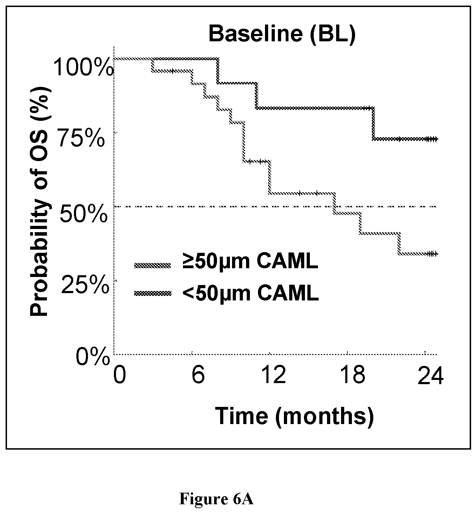

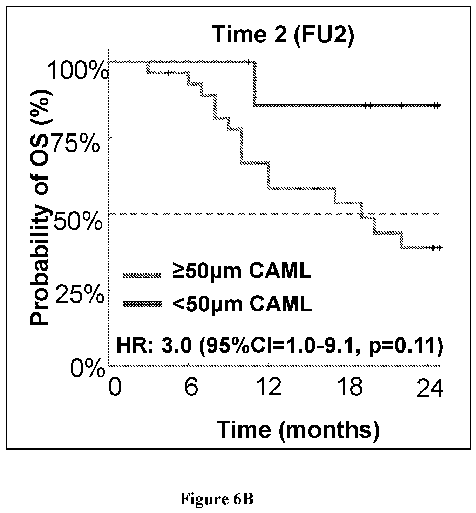

The overall clinical data are:
- CAMLs were found in 97% of cancer patients at BL, 97% at FU1 and 94% at FU2
- Over two years, seven patients showed no clinical disease progression (blue or upper line in **Figs. 5** and **6**), while 29 patients had observable clinical disease progression (red or lower line in **Figs. 5** and **6**).
- Of the patients with no progression (blue or upper line in **Figs. 5** and **6****,** n=7), one had CAMLs of ≥ 50µm at all time points while six had only small CAMLs at all time points.
- Of the 29 patients that progressed,
   o 22 patients had ≥ 50µm CAMLs at all time points
   ∘ 5 patients had < 50µm CAMLs at BL which increased in size by FU2
   ∘ 1 patient had ≥ 50µm CAMLs at BL that decreased by FU2
   ∘ 1 patient had small CAMLs at all time points.

Whether CAML size can predict treatment response independent of type of solid tumor and type of treatments was evaluated.

**Figures 5A-5B** show the Kaplan Meier plots of progression free survival (PFS) at two time points: baseline (BL) **(****FIG. 5A****),** and second follow up (FU2) **(****FIG. 5B****).** The data is analyzed based on the size of the CAMLs, < 50 µm (blue (upper) curves) or ≥ 50 µm (red (lower) curves).

**Figures 6A-6B** show the Kaplan Meier plots of overall survival (OS) at two time points: baseline (BL) **(****FIG. 6A****),** and second follow up (FU2) **(****FIG. 6B****).** The data is analyzed based on the size of the CAMLs, < 50 µm (blue (upper) curves) or ≥ 50 µm (red (lower) curves).

We show that increased CAML enlargement compared to baseline indicates not responding to treatment, resulting in shorter PFS in a variety of cancer types.

### Study 5: Combining CTC and CAML Data

Another set of results are related to different cancers and different treatments. A prospective two year blind multi-institutional study was undertaken to evaluate the inclusion of CTCs in addition to CAMLs size before, and after, induction of a new line of therapy. Total n=91 patients were recruited: breast (n = 14), esophageal (n = 23), NSCLC (n = 23), prostate (n = 21), and small cell lung cancer (SCLC) (n = 10) in Stage III (n = 53) or Stage IV (n =38) disease. A baseline (BL) blood sample was taken prior to induction of a new therapy and a single follow up (FU) taken after initiation of systemic therapy (~30 days). Blood was filtered by CellSieve filtration. The quantities and subtypes of CTCs & CAMLs were analyzed based on OS hazard ratios (HRs) by censored univariate & multivariate analysis.

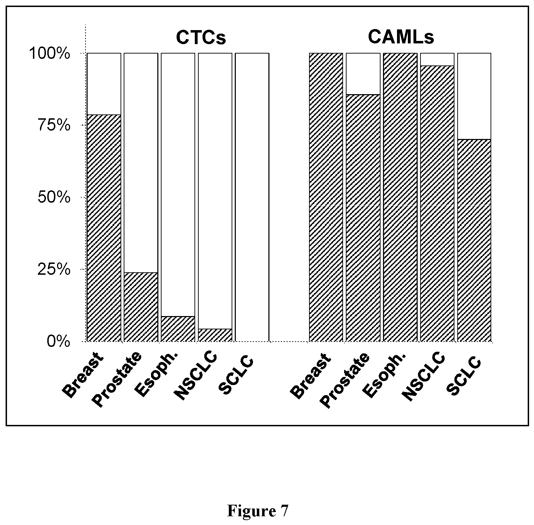

**Figure 7** shows CTCs were rare in lung (6%), esophageal (4%) and prostate (24%), but common in breast (79%). CAMLs were common in all the cancers, found in 92% of BL samples and 98% of FU samples with both times being prognostic for OS.

CTCs were identified in 21% of patients at BL, with a single CTC being prognostic for OS (HR = 2.4 95%CI=1.1-5.1, p = 0.048). Further, CTCs were found in 23% of samples at FU and also prognostic for OS (HR = 3.1 95%CI=1.4-6.9, p = 0.013). However, CTCs were rare in lung (6%), esophageal (4%) and prostate cancers (24%), but common in breast (79%). In contrast, CAMLs were found in 92% of BL, with ≥50µm CAMLs being a prognostic for OS (HR = 3.0, 95% CI=1.6-.5.7, p = 0.001). At FU, CAMLs were found in 98% of FU samples, the OS prognostic value of ≥50µm CAMLs increased (HR = 3.5 95% CI=1.9-6.6, p = 0.001). Further, after induction of systemic therapy, the presence of both, >1 CTCs or a ≥ 50µm CAML was 75% accurate at predicting survival of patients within 24 months, with OS HR = 3.7 95%CI=2.0-7.0, p = 0.001.

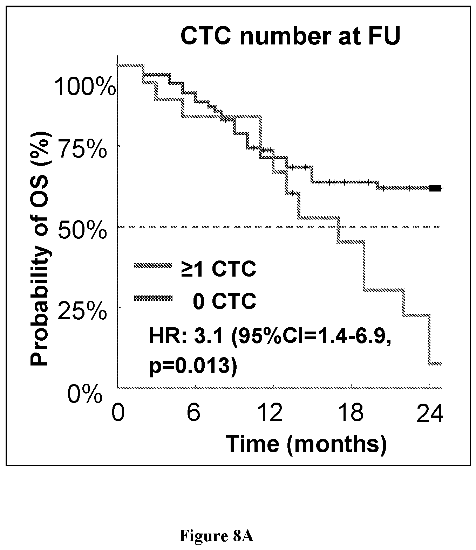

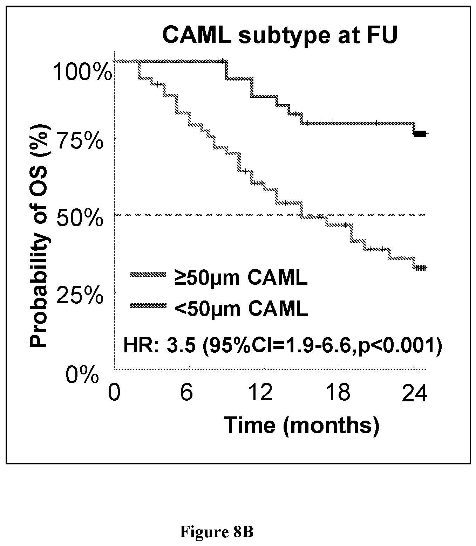

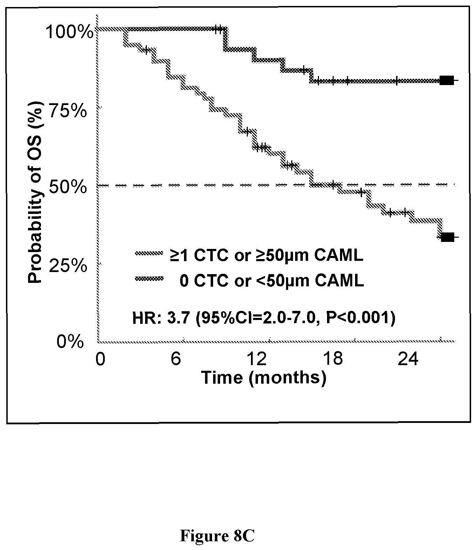

At FU, a single CTC **(****FIG. 8A****)** was associated with poorer OS (lower (red) curve); a single CAML ≥50µm CAML **(****FIG. 8B****)** was also associated with poorer OS (lower (red) curve). The OS of simultaneous measurement of both CTCs and CAMLs by blood test is presented in **FIG. 8C** with the single CAML and CTC shown as the lower (red) curve.

This data suggests that simultaneous measurement of both CTCs and CAML size may increase the prognostic value of blood based diagnostics and may be predictive of benefit of subsequent therapies.

This data suggest that CAML size and changes of CAML size is applicable to predicting treatment response, or disease progression for major solid tumors.

### Example 2

### Cancer Vaccine

Cancer vaccines can be administered to a subject in the form of cells expressing the markers intended as vaccine targets. The cells can be in the form of cancer cells, modified viruses and other types of modified cells. After the subject receives the vaccine, the body's immune system produces T cells that recognize and attack antigens expressed by the vaccine as well as cancer cells.

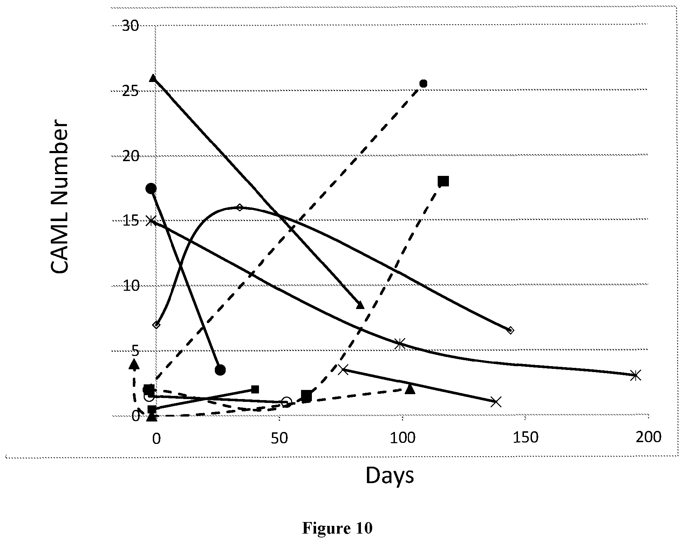

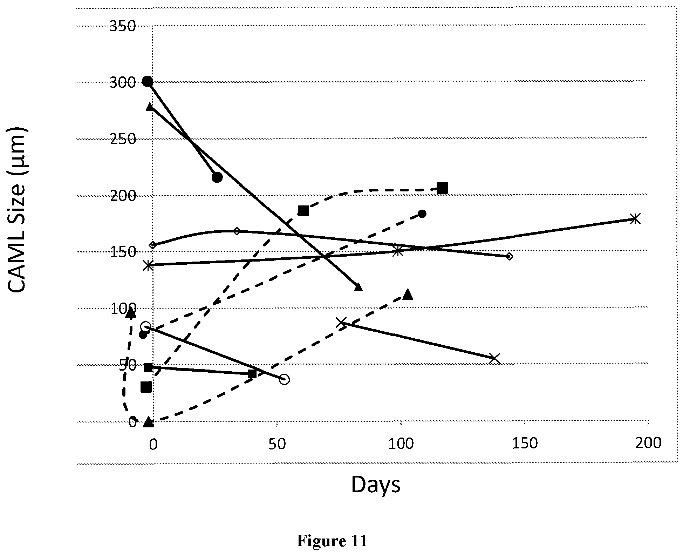

The data to support the concept of utilizing CAMLs to monitor and/or predict patient treatment response for a cancer vaccine is based on ten breast cancer patients treated with the SV-BR-1-GM vaccine. 7.5 mL of blood were taken and analyzed at different time points. The data presented in **Fig. 10** and **Fig. 11** show CAML data for patients expressing at least one HLA allele (solid curves) and patients not expressing any HLA allele (dashed curves).

**Figure 10** shows numbers of CAMLs during treatment. The patients expressing at least one HLA allele have a reduced number or fewer CAMLs during the treatment, indicating a positive response. For one patient, the patient with a data point at 200 day, the lung and the soft tissue metastasis were completely eliminated. The patients not expressing HLA alleles do not appear to benefit from the vaccine and the CAML numbers were found to be increased.

**Figure 11** shows the size of CAMLs during treatment. All the patients except one expressing at least one HLA allele exhibited a reduction in CAML size during the treatment. However, CAML sizes for most patients were still much larger than 50 µm, indicating the presence of an aggressive cancer. Imaging of the patients demonstrated that they all still had cancer (data not shown).

The change in CAML number is applicable to other treatments and other cancers besides vaccines, especially for cancers that typically have more than five CAMLs in subjects having Stage IV cancer.

### Example 3

CAMLs are circulating stromal cells common in the peripheral blood of cancer patients hypothesized to be a mechanism in cancer pathogenesis. Presented here are the results of a prospective study on treatment naive lung cancer patients before induction and directly after completion of definitive radiotherapy to determine if CAMLs are predictive of treatment response and cancer progression.

Methods: a two-year single blind prospective study was undertaken, testing the relationship of enlarged CAMLs (≥50µm) to progression free survival (PFS) of lung cancer patients before and after induction of definitive radiation therapy. To achieve a 2-tailed 95% power (α=0.05), a training set of 55 patients was recruited, all with pathologically confirmed lung cancer: Stage I (n=13), Stage II (n=7), Stage IIIa (n=10), Stage IIIb (n=18) & Stage IV (n=7). Baseline (BL) blood samples were taken prior to start of therapy. If possible, a second blood sample (T1) was taken after completion of radiotherapy (~60 days), n=46 patients. Blood was filtered by CellSieve^{™} filtration and CAMLs quantified. Analysis by CAML size of <50 µm or ≥50 µm was used to evaluate PFS hazard ratios (HRs) by censored univariate & multivariate analyses.

Results (data not shown): CAMLs were found in 93% of BL samples averaging 3.2 CAMLs/7.5mL. Patients with at least one CAML ≥50 µm had reduced PFS (HR=2.9, 95%CI 1.4-6.0, p=0.010). 46 patients agreed to a follow up blood draw. 13 of the 46 patients had an increase of CAML size to ≥50 µm, but 3 patients had a decrease to <50 µm, resulting in an increase of PFS (HR=7.7, 95%CI 2.6-12.5, p<0.001). Combined, 90% of patients with a ≥50 µm CAML at BL progressed within 2 years vs 46% of patients with <50 µm CAMLs. Enlarged CAMLs at T1 was more predictive of progression, with 92% of patients with a ≥50 µm CAML progressing vs 21% of patients with <50 µm CAMLs. Notably, 100% of patients that had a ≥50 µm CAML at both BL and T1 progressed. By comparison, only 11% of patients with <50 µm CAMLs at both BL and T1 progressed. In a multivariate analysis, CAML size was the most significant indicator of PFS and OS, independent of all other clinical variables, including stage.

This data suggests that for lung cancer patients undergoing definitive radiation therapy, the presence of enlarged CAMLs appears to predict patients that are resistant to treatment. Furthermore, these circulating stromal cells appear useful for sequential monitoring of patients that may prognosticate who are likely to progress after treatment.

### CITATIONS

1. Adams, D., et al., Circulating giant macrophages as a potential biomarker of solid tumors. PNAS 2014, 111(9):3514-3519.
2. International Patent Application Publication No. WO 2013/181532, dated December 5, 2013.
3. Adams, D. L., et al., Cytometric characterization of Circulating Tumor Cells captured by microfiltration and their correlation to the CellSearch® CTC test. Cytometry Part A 2015; 87A:137-144.
4. Adams DL, et al. The systematic study of circulating tumor cell isolation using lithographic microfilters. RSC Adv 2014, 4:4334-4342.
5. International Patent Application Publication No. WO 2013/078409, dated May 30, 2013.
6. International Patent Application Publication No. WO 2016/33103, dated March 3, 2016.
7. Adams D.L., et al., Mitosis in Circulating Tumor Cells stratifies highly aggressive breast carcinomas, Breast Cancer Research 2016; 18:44.

## Claims

1. A method for predicting cancer progression in a subject having cancer comprising determining the size of circulating cancer associated macrophage-like cells (CAMLs) in a biological sample obtained from a subject having cancer, and making a prediction based thereon, wherein when each CAML in the sample is less than about 50 µm in size, the cancer is predicted not to progress, and wherein when at least one CAML in the sample is about 50 µm or more in size, the cancer is predicted to progress.

2. A method for predicting cancer progression in a subject having a cancer comprising determining the size of CAMLs in a first and second biological sample, and optional additional biological samples, obtained from a subject having cancer, wherein the first sample is obtained from the subject prior to or during cancer treatment, wherein the second sample and optional additional samples are obtained from the subject after at least one cancer treatment,
wherein when the average size of CAMLs in the second and optional additional samples is decreased in comparison to the average size of the CAMLs in the first sample, the cancer is predicted not to progress in the subject; or
wherein when the average size of CAMLs in the second and optional additional samples is maintained or increased in comparison to the average size of the CAMLs in the first sample, the cancer is predicted to progress in the subject; or
wherein when the size of at least one CAML in the first sample is about 50 µm or more, and the size of each cell in the second and optional additional samples is less than about 50 µm, the cancer is predicted not to progress; or
wherein when the size of each CAML in the first sample is less than about 50 µm, and when at least one CAML in the second and optional additional samples is greater than about 50 µm in size, the cancer is predicted to progress.

3. A method for predicting cancer progression in a subject having a cancer comprising determining the number of CAMLs in a first and second biological sample, and optional additional biological samples, obtained from a subject having cancer, wherein the first sample is obtained from the subject prior to or during cancer treatment, wherein the second sample and optional additional samples are obtained from the subject after at least one cancer treatment, wherein when the number of CAMLs in the second and optional additional samples is decreased in comparison to the number of the CAMLs in the first sample, the cancer is predicted not to progress, and wherein when the number of CAMLs in the second and optional additional samples is maintained or increased in comparison to the number of the CAMLs in the first sample, the cancer is predicted to progress.

4. A method for predicting response to treatment of a subject having cancer comprising determining the size of CAMLs in a biological sample obtained from the subject, and making a prediction based thereon, wherein when each CAML in the sample is less than about 50 µm in size, the subject is predicted to respond to treatment, and wherein when at least one CAML in the sample is about 50 µm or more in size, the subject is predicted to not respond to treatment.

5. A method for predicting response to treatment of a subject having cancer comprising determining the size of CAMLs in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, and
wherein when the average size of CAMLs in the second and optional additional samples is decreased in comparison to the average size of the CAMLs in the first sample, the subject is predicted to respond to treatment;
wherein when the average size of CAMLs in the second and optional additional samples is maintained or increased in comparison to the average size of the CAMLs in the first sample, the subject is predicted to not respond to treatment;
wherein when the number of CAMLs greater than about 50 µm in size is maintained or increases from the first to the second and optional additional samples, the subject is predicted to not respond to treatment; or
wherein when the number of CAMLs greater than about 50 µm in size decreases from the first to the second and optional additional samples, the subject is predicted to respond to treatment; or
wherein when the size of each CAML in the second or following sample decreases in comparison to the first sample, and wherein the size of each CAML in the second or optional additional sample is less than about 50 µm, the subject is predicted to respond to treatment and potentially cured of cancer.

6. A method for predicting response to treatment of a subject having cancer comprising determining the absence of any CAMLs larger than about 50 µm in a biological sample from the subject and predicting the subject is responding to treatment.

7. A method for predicting response to treatment of a subject having cancer comprising determining the size of CAMLs in a biological sample obtained from a subject having cancer, wherein the sample is obtained from the subject after at least one cancer treatment, and wherein when each CAML is about 50 µm or less in size, the subject is predicted to respond to treatment.

8. A method for predicting response to treatment of a subject having cancer comprising determining the number of CAMLs in a first and second biological sample, and optional additional biological samples, obtained from a subject having cancer, wherein the first sample is obtained from the subject prior to or during cancer treatment, wherein the second and optional additional samples are obtained from the subject after at least one cancer treatment, wherein when the number of CAMLs decreases from the first to the second and optional additional samples, the subject is predicted to respond to treatment, and wherein when the number of CAMLs is maintained or increases from the first to the second and optional additional samples, the subject is predicted to not respond to treatment.

9. The method of any one of claims 1-8, wherein the CAMLs have the following characteristics:
(a) multiple individual nuclei and/or one or more fused nuclei having a size of about 14-64 µm;
(b) cell size of about 20-300 µm in size; and
(c) morphological shape selected from the group consisting of spindle, tadpole, round, oblong, two legs, more than two legs, thin legs, and amorphous, and
wherein the CAMLs have one or more of the following additional characteristics:
(d) CD14 positive phenotype;
(e) CD45 expression;
(f) EpCAM expression;
(g) vimentin expression;
(h) PD-L1 expression;
(i) monocytic CD11C marker expression;
(j) endothelial CD146 marker expression;
(k) endothelial CD202b marker expression;
(l) endothelial CD31 marker expression; and
(m) epithelial cancer cell CK8, 18, and/or 19 marker expression.

10. The method of any one of claims 1-8, wherein the size of the biological sample is between 5 and 15 mL, and wherein the source of the biological sample is one or more of peripheral blood, blood, lymph node, bone marrow, cerebral spinal fluid, tissue, urine, peripheral blood mononuclear cells (PBMCs), and cryopreserved PBMCs and wherein when the biological sample is blood, the blood is antecubital-vein blood, inferior-vena-cava blood, femoral vein blood, portal vein blood, or jugular-vein blood.

11. The method of any one of claims 1-8, wherein the cancer is a solid tumor, Stage I cancer, Stage II cancer, Stage III cancer, Stage IV cancer, carcinoma, sarcoma, neuroblastoma, melanoma, epithelial cell cancer, breast cancer, prostate cancer, lung cancer, pancreatic cancer, colorectal cancer, liver cancer, head and neck cancer, kidney cancer, ovarian cancer, esophageal cancer or other solid tumor cancer.

12. The method of any one of claims 1-8, wherein CAMLs are isolated from the biological sample using one or more means selected from the group consisting of size exclusion methodology, immunocapture, dendrimer-mediated multivalent cell capture, affinity based surface capture, biomimetic surface coating capture, selectin coated surfaces capture, other functionalized surface captures, inertial focusing chips, red blood cell lysis, white blood cell depletion, FICOLL separation, electrophoresis, dielectrophoresis, flow cytometry, magnetic levitation, and various microfluidic chips, or a combination thereof.

13. The method of claim 12, wherein circulating cells are isolated from the biological samples using size exclusion methodology that comprises using a microfilter, wherein the microfilter has a pore size ranging from about 5 µm to about 20 µm wherein the pores of the microfilter have a round, race-track shape, oval, square and/or rectangular pore shape.

14. The method of claim 12, wherein circulating cells are isolated using a microfluidic chip via physical size-based sorting, hydrodynamic size-based sorting, grouping, trapping, immunocapture, concentrating large cells, or eliminating small cells based on size.

15. The method of claim 1, wherein the subject is undergoing treatment, wherein the treatment is one or more of chemotherapy, single drug, combination of drugs, immunotherapy, radiation therapy, chemoradiation, radiation combined with single or multiple drug, chemoradiation combined with single or multiple drugs, cancer vaccine, and cell therapy.

16. The method of claim 15, wherein the treatment is a cancer vaccine and the subject expresses at least one HLA allele.

## Patentansprüche

1. Verfahren zum Vorhersagen des Fortschreitens von Krebs in einem Subjekt mit Krebs umfassend Bestimmen der Größe von zirkulierenden Krebs-assoziierten Makrophagenähnlichen Zellen (CAMLs) in einer biologischen Probe, welche von einem Subjekt mit Krebs erhalten wird, und Machen einer Vorhersage basierend darauf, wobei, wenn jede CAML in der Probe eine Größe von kleiner als etwa 50 µm aufweist, vorhergesagt wird, dass der Krebs nicht fortschreitet, und wobei, wenn mindestens eine CAMI, in der Probe eine Größe von etwa 50 µm oder größer aufweist, vorhergesagt wird, dass der Krebs fortschreitet.

2. Verfahren zum Vorhersagen des Fortschreitens von Krebs in einem Subjekt mit einem Krebs umfassend Bestimmen der Größe von CAMLs in einer ersten und zweiten biologischen Probe, und optionalen zusätzlichen biologischen Proben, welche von einem Subjekt mit Krebs erhalten werden, wobei die erste Probe von dem Subjekt vor oder während Krebsbehandlung erhalten wird, wobei die zweite Probe und optionale zusätzliche Proben von dem Subjekt nach mindestens einer Krebsbehandlung erhalten werden,
wobei, wenn die mittlere Größe von CAMLs in der zweiten und optionalen zusätzlichen Proben im Vergleich zur mittleren Größe der CAMLs in der ersten Probe abgenommen hat, vorhergesagt wird, dass der Krebs in dem Subjekt nicht fortschreitet; oder
wobei, wenn die mittlere Größe von CAMLs in der zweiten und optionalen zusätzlichen Proben im Vergleich zur mittleren Größe der CAMLs in der ersten Probe gleichgeblieben ist oder zugenommen hat, vorhergesagt wird, dass der Krebs in dem Subjekt fortschreitet; oder
wobei, wenn die Größe von mindestens einer CAML in der ersten Probe etwa 50 µm oder mehr beträgt, und die Größe von jeder Zelle in der zweiten und optionalen zusätzlichen Proben weniger als etwa 50 µm beträgt, vorhergesagt wird, dass der Krebs nicht fortschreitet; oder
wobei, wenn die Größe von jeder CAML in der ersten Probe weniger als etwa 50 µm beträgt, und wenn mindestens eine CAML in der zweiten und optionalen zusätzlichen Proben eine Größe von größer als etwa 50 µm aufweist, vorhergesagt wird, dass der Krebs fortschreitet.

3. Verfahren zum Vorhersagen des Fortschreitens von Krebs in einem Subjekt mit einem Krebs umfassend Bestimmen der Anzahl von CAMLs in einer ersten und zweiten biologischen Probe, und optionalen zusätzlichen biologischen Proben, welche von einem Subjekt mit Krebs erhalten werden, wobei die erste Probe von dem Subjekt vor oder während Krebsbehandlung erhalten wird, wobei die zweite Probe und optionale zusätzliche Proben von dem Subjekt nach mindestens einer Krebsbehandlung erhalten werden, wobei, wenn die Anzahl von CAMLs in der zweiten und optionalen zusätzlichen Proben im Vergleich zur Anzahl der CAMLs in der ersten Probe abgenommen hat, vorhergesagt wird, dass der Krebs nicht fortschreitet, und wobei, wenn die Anzahl von CAMLs in der zweiten und optionalen zusätzlichen Proben im Vergleich zur Anzahl der CAMLs in der ersten Probe gleichgeblieben ist oder zugenommen hat, vorhergesagt wird, dass der Krebs fortschreitet.

4. Verfahren zum Vorhersagen einer Reaktion auf Behandlung eines Subjekts mit Krebs umfassend Bestimmen der Größe von CAMLs in einer biologischen Probe, welche von dem Subjekt erhalten wird, und Machen einer Vorhersage basierend darauf, wobei, wenn jede CAML in der Probe eine Größe von kleiner als etwa 50 µm aufweist, vorhergesagt wird, dass das Subjekt auf Behandlung reagiert, und wobei, wenn mindestens eine CAML in der Probe eine Größe von etwa 50 µm oder größer aufweist, vorhergesagt wird, dass das Subjekt nicht auf Behandlung reagiert.

5. Verfahren zum Vorhersagen einer Reaktion auf Behandlung eines Subjekts mit Krebs umfassend Bestimmen der Größe von CAMLs in einer ersten und zweiten biologischen Probe, und optionalen zusätzlichen biologischen Proben, welche von einem Subjekt mit Krebs erhalten werden, wobei die erste Probe von dem Subjekt vor oder während Krebsbehandlung erhalten wird, wobei die zweite und optionale zusätzliche Proben von dem Subjekt nach mindestens einer Krebsbehandlung erhalten werden, und
wobei, wenn die mittlere Größe von CAMLs in der zweiten und optionalen zusätzlichen Proben im Vergleich zur mittleren Größe der CAMLs in der ersten Probe abgenommen hat, vorhergesagt wird, dass das Subjekt auf Behandlung reagiert;
wobei, wenn die mittlere Größe von CAMLs in der zweiten und optionalen zusätzlichen Proben im Vergleich zur mittleren Größe der CAMLs in der ersten Probe gleichgeblieben ist oder zugenommen hat, vorhergesagt wird, dass das Subjekt nicht auf Behandlung reagiert;
wobei, wenn die Anzahl von CAMLs, die eine Größe von größer als etwa 50 µm aufweisen, von der ersten zu der zweiten und optionalen zusätzlichen Proben gleichbleibt oder zunimmt, vorhergesagt wird, dass das Subjekt nicht auf Behandlung reagiert; oder
wobei, wenn die Anzahl von CAMLs, die eine Größe von größer als etwa 50 µm aufweisen, von der ersten zu der zweiten und optionalen zusätzlichen Proben abnimmt, vorhergesagt wird, dass das Subjekt auf Behandlung reagiert; oder
wobei, wenn die Größe von jeder CAML in der zweiten oder folgenden Probe im Vergleich zur ersten Probe abnimmt, und wobei die Größe von jeder CAML in der zweiten oder optionalen zusätzlichen Probe weniger als etwa 50 µm beträgt, vorhergesagt wird, dass das Subjekt auf Behandlung reagiert und möglicherweise von Krebs geheilt ist.

6. Verfahren zum Vorhersagen einer Reaktion auf Behandlung eines Subjekts mit Krebs umfassend Bestimmen der Abwesenheit von jedweden CAMLs, welche größer als etwa 50 µm sind, in einer biologischen Probe von dem Subjekt und Vorhersagen, dass das Subjekt auf Behandlung reagiert.

7. Verfahren zum Vorhersagen einer Reaktion auf Behandlung eines Subjekts mit Krebs umfassend Bestimmen der Größe von CAMLs in einer biologischen Probe, welche von einem Subjekt mit Krebs erhalten wird, wobei die Probe von dem Subjekt nach mindestens einer Krebsbehandlung erhalten wird, und wobei, wenn jede CAML eine Größe von etwa 50 µm oder kleiner aufweist, vorhergesagt wird, dass das Subjekt auf Behandlung reagiert.

8. Verfahren zum Vorhersagen einer Reaktion auf Behandlung eines Subjekts mit Krebs umfassend Bestimmen der Anzahl von CAMLs in einer ersten und zweiten biologischen Probe, und optionalen zusätzlichen biologischen Proben, welche von einem Subjekt mit Krebs erhalten werden, wobei die erste Probe von dem Subjekt vor oder während Krebsbehandlung erhalten wird, wobei die zweite und optionale zusätzliche Proben von dem Subjekt nach mindestens einer Krebsbehandlung erhalten werden, wobei, wenn die Anzahl von CAMLs von der ersten zu der zweiten und optionalen zusätzlichen Proben abnimmt, vorhergesagt wird, dass das Subjekt auf Behandlung reagiert, und wobei, wenn die Anzahl von CAMLs von der ersten zu der zweiten und optionalen zusätzlichen Proben gleichgeblieben ist oder zugenommen hat, vorhergesagt wird, dass das Subjekt nicht auf Behandlung reagiert.

9. Verfahren nach einem der Ansprüche 1-8, wobei die CAMLs die folgenden Charakteristika aufweisen:
(a) multiple einzelne Kerne und/oder einen oder mehr verschmolzene Kerne mit einer Größe von etwa 14-64 µm;
(b) Zellengröße von etwa 20-300 µm; und
(c) morphologische Gestalt ausgewählt aus der Gruppe bestehend aus Spindel, Kaulquappe, kugelförmig, länglich, zwei Beine, mehr als zwei Beine, dünne Beine, und amorph, und
wobei die CAMLs eines oder mehr der folgenden zusätzlichen Charakteristika aufweisen:
(d) CD14-positiver Phänotyp;
(e) CD45-Expression;
(f) EpCAM-Expression;
(g) Vimentin-Expression;
(h) PD-L1-Expression;
(i) monozytischer CD11C-Marker-Expression;
(j) endothelialer CD146-Marker-Expression;
(k) endothelialer CD202b-Marker-Expression;
(l) endothelialer CD31-Marker-Expression; und
(m)epithelialer Krebszelle CK8, 18, und/oder 19-Marker-Expression.

10. Verfahren nach einem der Ansprüche 1-8, wobei die Größe der biologischen Probe zwischen 5 und 15 mL liegt, und wobei die Quelle der biologischen Probe eine oder mehr von peripherem Blut, Blut, Lymphknoten, Knochenmark, Zerebrospinalflüssigkeit, Gewebe, Urin, peripheren mononukleären Blutzellen (PBMCs), und kryokonservierten PBMCs ist und wobei, wenn die biologische Probe Blut ist, das Blut Antekubitalvene-Blut, untere Hohlvene-Blut, Oberschenkelvene-Blut, Pfortader-Blut, oder Jugularvene-Blut ist.

11. Verfahren nach einem der Ansprüche 1-8, wobei der Krebs ein solider Tumor, Stadium I-Krebs, Stadium II-Krebs, Stadium III-Krebs, Stadium IV-Krebs, Karzinom, Sarkom,
Neuroblastom, Melanom, Epithelzellkrebs, Brustkrebs, Prostatakrebs, Lungenkrebs, Bauchspeicheldrüsenkrebs, Kolorektalkrebs, Leberkrebs, Kopf und Nacken-Krebs, Nierenkrebs, Eierstockkrebs, Speiseröhrenkrebs oder ein anderer solider Tumor-Krebs ist.

12. Verfahren nach einem der Ansprüche 1-8, wobei CAMLs von der biologischen Probe isoliert werden unter Verwendung von einem oder mehr Mitteln ausgewählt aus der Gruppe bestehend aus Größenausschluss-Methodik, Immuneinfang, Dendrimervermitteltem multivalente Zelle-Einfang, Oberflächeneinfang auf Affinitäts-Basis, biomimetischer Oberflächenüberzug-Einfang, mit Selektin überzogene OberflächenEinfang, anderen funktionalisierte Oberfläche-Einfängen, auf Trägheit fokussierten Chips, Lyse von roten Blutkörperchen, Depletion von weißen Blutkörperchen, FICOLL-Trennung, Elektrophorese, Dielektrophorese, Durchflusszytometrie, magnetischem Schweben, und verschiedenen Mikrofluid-Chips, oder einer Kombination davon.

13. Verfahren nach Anspruch 12, wobei zirkulierende Zellen von den biologischen Proben unter Verwendung von Größenausschluss-Methodik, welche Verwenden eines Mikrofilters umfasst, isoliert werden, wobei das Mikrofilter eine Porengröße in einem Bereich von etwa 5 µm bis etwa 20 µm aufweist, wobei die Poren des Mikrofilters eine runde, Rennbahn-förmige, ovale, quadratische und/oder rechteckige Porenform aufweisen.

14. Verfahren nach Anspruch 12, wobei zirkulierende Zellen unter Verwendung eines Mikrofluid-Chips über physikalisches Sortieren auf Größen-Basis, hydrodynamisches Sortieren auf Größen-Basis, Gruppenbestimmung, Trapping, Immuneinfang, Konzentrieren von großen Zellen, oder Eliminieren von kleinen Zellen auf der Basis von Größe isoliert werden.

15. Verfahren nach Anspruch 1, wobei sich das Subjekt einer Behandlung unterzieht, wobei die Behandlung eine oder mehr von Chemotherapie, einzelnem Arzneistoff, Kombination von Arzneistoffen, Immuntherapie, Strahlentherapie, Chemoradiotherapie, Strahlung kombiniert mit einem einzelnen oder mehreren Arzneistoffen, Chemoradiotherapie kombiniert mit einem einzelnen oder mehreren Arzneistoffen, Krebsimpfstoff, und Zelltherapie ist.

16. Verfahren nach Anspruch 15, wobei die Behandlung ein Krebsimpfstoff ist und das Subjekt mindestens ein HLA-Allel exprimiert.

## Revendications

1. Procédé de prédiction de la progression d'un cancer chez un sujet atteint de cancer comprenant la détermination de la taille de cellules cancéreuses circulantes semblables à des macrophages associés aux tumeurs (CAML : cancer associated macrophage-like) dans un échantillon biologique prélevé sur un sujet atteint de cancer, et la réalisation d'une prédiction à partir de celle-ci, dans lequel quand chaque cellule CAML de l'échantillon présente une taille inférieure à 50 µm environ, on prédit l'absence de progression du cancer, et dans lequel quand au moins une cellule CAML de l'échantillon présente une taille supérieure ou égale à environ 50 µm, on prédit que le cancer a progressé.

2. Procédé de prédiction de la progression d'un cancer chez un sujet atteint de cancer comprenant la détermination de la taille de cellules CAML dans un premier et un deuxième échantillons biologiques, et des échantillons biologiques supplémentaires optionnels, prélevés sur un sujet atteint de cancer, dans lequel le premier échantillon est prélevé sur le sujet avant ou pendant un traitement du cancer, dans lequel le deuxième échantillon et les échantillons supplémentaires optionnels sont prélevés sur le sujet après au moins un traitement du cancer,
dans lequel si la taille moyenne des cellules CAML du deuxième échantillon et des échantillons supplémentaires optionnels a diminué par rapport à la taille moyenne des cellules CAML du premier échantillon, on prédit l'absence de progression du cancer chez le sujet ; ou
dans lequel quand la taille moyenne des cellules CAML du deuxième échantillon et des échantillons supplémentaires optionnels se maintient ou a augmenté par rapport à la taille moyenne des cellules CAML du premier échantillon, on prédit que le cancer a progressé chez le sujet ; ou
dans lequel quand la taille d'au moins une cellule CAML du premier échantillon est supérieure ou égale à environ 50 µm ou la taille de chaque cellule du deuxième échantillon et des échantillons supplémentaires optionnels est inférieure à environ 50 µm, on prédit l'absence de progression du cancer ; ou
dans lequel quand la taille de chaque cellule CAML du premier échantillon est inférieure à environ 50 µm, et quand la taille d'au moins une cellule CAML du deuxième échantillon et des échantillons supplémentaires optionnels est supérieure à environ 50 µm, on prédit que le cancer a progressé.

3. Procédé de prédiction de la progression d'un cancer chez un sujet atteint de cancer comprenant la détermination du nombre de cellules CAML dans un premier et un deuxième échantillons biologiques, et des échantillons supplémentaires optionnels, prélevés sur un sujet atteint de cancer, dans lequel le premier échantillon est prélevé sur le sujet avant ou pendant un traitement du cancer, dans lequel le deuxième échantillon et les échantillons supplémentaires optionnels sont prélevés sur le sujet après au moins un traitement du cancer, dans lequel quand le nombre de cellules CAML du deuxième échantillon et des échantillons supplémentaires optionnels a diminué par rapport au nombre de cellules CAML du premier échantillon, on prédit l'absence de progression du cancer et dans lequel quand le nombre de cellules CAML du deuxième échantillon et des échantillons supplémentaires optionnels se maintient ou a augmenté par rapport au nombre de cellules CAML du premier échantillon, on prédit que le cancer a progressé.

4. Procédé de prédiction de la réponse à un traitement d'un sujet atteint de cancer comprenant la détermination de la taille des cellules CAML dans un échantillon biologique prélevé sur le sujet, et la réalisation d'une prédiction à partir de celle-ci, dans lequel quand chaque cellule CAML de l'échantillon présente une taille inférieure à environ 50 µm, on prédit que le sujet répond au traitement, et dans lequel quand au moins une cellule CAML de l'échantillon présente une taille d'environ 50 µm ou plus, on prédit l'absence de réponse du sujet au traitement.

5. Procédé de prédiction de la réponse à un traitement d'un sujet atteint de cancer comprenant la détermination de la taille des cellules CAML dans un premier et un deuxième échantillons biologiques, et des échantillons biologiques supplémentaires optionnels, prélevés sur un sujet atteint de cancer, dans lequel le premier échantillon est prélevé sur le sujet avant ou pendant un traitement du cancer, dans lequel le deuxième échantillon et les échantillons supplémentaires optionnels sont prélevés sur le sujet après au moins un traitement du cancer, et
dans lequel quand la taille moyenne des cellules CAML du deuxième échantillon et des échantillons supplémentaires optionnels a diminué par rapport à la taille moyenne des cellules CAML du premier échantillon, on prédit que le sujet répond au traitement ;
dans lequel quand la taille moyenne des cellules CAML du deuxième échantillon et des échantillons supplémentaires optionnels se maintient ou a augmenté par rapport à la taille moyenne des cellules CAML du premier échantillon, on prédit l'absence de réponse du sujet au traitement ;
dans lequel quand le nombre de cellules CAML présentant une taille supérieure à environ 50 µm se maintient ou a augmenté du premier au deuxième échantillon et aux échantillons supplémentaires optionnels, on prédit l'absence de réponse du sujet au traitement ; ou
dans lequel quand le nombre de cellules CAML présentant une taille supérieure à environ 50 µm diminue du premier au deuxième échantillon et aux échantillons supplémentaires optionnels, on prédit que le sujet répond au traitement ; ou
dans lequel quand la taille de chaque cellule CAML du deuxième échantillon ou de l'échantillon suivant a diminué par rapport au premier échantillon, et dans lequel la taille de chaque cellule CAML du deuxième échantillon ou des échantillons supplémentaires optionnels est inférieure à environ 50 µm, on prédit que le sujet répond au traitement et est potentiellement guéri du cancer.

6. Procédé de prédiction de la réponse à un traitement d'un sujet atteint de cancer comprenant la détermination de l'absence de toute cellule CAML de taille supérieure à environ 50 µm dans un échantillon biologique du sujet et la prédiction que le sujet répond au traitement.

7. Procédé de prédiction de la réponse à un traitement d'un sujet atteint de cancer comprenant la détermination de la taille des cellules CAML dans un échantillon biologique prélevé sur un sujet atteint de cancer, dans lequel l'échantillon est prélevé sur le sujet après au moins un traitement du cancer, et dans lequel quand chaque cellule CAML présente une taille inférieure ou égale à environ 50 µm, on prédit que le sujet répond au traitement.

8. Procédé de prédiction de la réponse à un traitement d'un sujet atteint de cancer comprenant la détermination du nombre de cellules CAML dans un premier et un deuxième échantillons biologiques, et des échantillons biologiques supplémentaires optionnels, prélevés sur un sujet atteint de cancer, dans lequel le premier échantillon est prélevé sur le sujet avant ou pendant un traitement du cancer, dans lequel le deuxième échantillon et les échantillons supplémentaires optionnels sont prélevés sur le sujet après au moins un traitement du cancer, dans lequel quand le nombre de cellules CAML diminue du premier au deuxième échantillon et aux échantillons supplémentaires optionnels, on prédit que le sujet répond au traitement, et dans lequel quand le nombre de cellules CAML se maintient ou augmente du premier au deuxième échantillon et aux échantillons supplémentaires optionnels, on prédit l'absence de réponse du sujet au traitement.

9. Procédé selon l'une quelconque des revendications 1 à 8, dans lequel les cellules CAML présentent les caractéristiques suivantes :
(a) noyaux individuels multiples et/ou un ou plusieurs noyaux fusionnés présentant une taille de 14 à 64 µm environ ;
(b) taille de cellule d'environ 20 à 300 µm ; et
(c) forme morphologique choisie dans le groupe consistant en les formes en fuseau, en têtard, ronde, oblongue, à deux jambes, à plus de deux jambes, à jambes fines et amorphe, et
dans lequel les cellules CAML présentent une ou plusieurs des caractéristiques supplémentaires suivantes :
(d) phénotype CD 14 positif;
(e) expression de CD45 ;
(f) expression d'EpCAM ;
(g) expression de vimentine ;
(h) expression de PD-L1 ;
(i) expression de marqueur de monocytes CD11C ;
(j) expression de marqueur endothélial CD146 ;
(k) expression de marqueur endothélial CD202b ;
(l) expression de marqueur endothélial CD31 ; et
(m) expression de marqueur de cellule cancéreuse épithéliale CK8, 18 et/ou 19.

10. Procédé selon l'une quelconque des revendications 1 à 8, dans lequel la taille de l'échantillon biologique est comprise entre 5 et 15 mL et dans lequel la source de l'échantillon biologique est du sang périphérique, du sang, un ganglion lymphatique, la moëlle épinière, du fluide cérébro-spinal, du tissu, de l'urine, des cellules mononucléées du sang périphérique (PBMC : peripheral blood mononuclear cells), et des cellules PBMC cryoconservées, ou plusieurs d'entre ceux-ci, et dans lequel quand l'échantillon biologique est du sang, le sang est du sang de veine antécubitale, du sang de veine cave inférieure, du sang de veine fémorale, du sang de veine porte ou du sang de veine jugulaire.

11. Procédé selon l'une quelconque des revendications 1 à 8, dans lequel le cancer est une tumeur solide, un cancer de stade I, un cancer de stade II, un cancer de stade III, un cancer de stade IV, un carcinome, un sarcome, un neuroblastome, un mélanome, un cancer des cellules épithéliales, un cancer du sein, un cancer de la prostate, un cancer du poumon, un cancer du pancréas, un cancer colorectal, un cancer du foie, un cancer des voies aérodigestives supérieures, un cancer du rein, un cancer de l'ovaire, un cancer de l'oesophage ou autre cancer à tumeurs solides.

12. Procédé selon l'une quelconque des revendications 1 à 8, dans lequel les cellules CAML sont isolées de l'échantillon biologique au moyen d'un ou plusieurs moyens choisis dans le groupe consistant en la méthodologie d'exclusion de taille, l'immunocapture, la capture de cellules multivalentes induite par dendrimères, la capture de surface fondée sur l'affinité, la capture par revêtement biomimétique de surface, la capture de surface revêtue de sélectine, d'autres captures de surfaces fonctionnalisées, les puces de focalisation inertielle, la lyse de globules rouges sanguins, la déplétion de globules blancs sanguins, la séparation FICOLL, l'électrophorèse, la diélectrophorèse, la cytométrie en flux, la lévitation magnétique et diverses puces microfluidiques ou leurs combinaisons.

13. Procédé selon la revendication 12, dans lequel les cellules circulantes sont isolées des échantillons biologiques au moyen d'une méthodologie d'exclusion de taille qui comprend l'utilisation d'un microfiltre, dans lequel le microfiltre présente une taille de pore comprise entre environ 5 µm et environ 20 µm, dans lequel les pores du microfiltre présentent une forme de pore ronde, en piste de course, ovale, carrée et/ou rectangulaire.

14. Procédé selon la revendication 12, dans lequel les cellules circulantes sont isolées au moyen d'une puce microfluidique par un tri sur des critères de taille physique, un tri sur des critères de taille hydrodynamique, un regroupement, un piégeage, une immunocapture, la concentration de cellules de grande taille ou de l'élimination de petites cellules sur des critères de taille.

15. Procédé selon la revendication 1, dans lequel le sujet suit un traitement, dans lequel le traitement est une ou plusieurs d'une chimiothérapie, d'un médicament unique, d'une combinaison de médicaments, d'une immunothérapie, d'une radiothérapie, d'une chimio-radiothérapie, des rayonnements combinés à un seul ou plusieurs médicaments, une chimio-radiothérapie combinée à un seul ou plusieurs médicaments, un vaccin anticancéreux et une thérapie cellulaire.

16. Procédé selon la revendication 15, dans lequel le traitement est un vaccin anticancéreux et le sujet exprime au moins un allèle HLA.
